# EUROPEAN PATENT SPECIFICATION

(11) **EP 1 547 544 B1**
(45) Date of publication and mention of the grant of the patent: **23.10.2013**
(21) Application number: 03792837.1
(22) Date of filing: 25.08.2003
(51) Int. Cl.: A61C 19/00, A61C 8/00

(54) **ARTIFICIAL TOOTH ROOT IMPLANTATION POSITION DETERMINING INSTRUMENT,**
INSTRUMENT ZUR BESTIMMUNG DER IMPLANTATIONSPOSITION EINER KÜNSTLICHEN ZAHNWURZEL
INSTRUMENT DE DÉTERMINATION DE LA POSITION D'IMPLANTATION DE RACINES DE DENTS ARTIFICIELLES

(30) Priority: 26.08.2002 JP 2002245499
(43) Date of publication of application: 29.06.2005
(73) Proprietor: iCAT CORPORATION, Osaka 532-0004 (JP)
(72) Inventor: SOGO, Motofumi c/o Osaka Univ. Dental Hospital, Suita-shi, Osaka 565-0871 (JP); MAEDA, Yoshinobu c/o Osaka Univ. Dental Hospital, Suita-shi, Osaka 565-0871 (JP); TSUTSUMI, Sadami c/o Dept. Medical Sim. Eng. Inst., Kyoto-shi, Kyoto 606-8507 (JP)
(74) Representative: Van den Heuvel, Henricus Theodorus
(86) International application number: PCT/JP2003/010719
(87) International publication number: WO 2004/017860

(56) References cited:
- WO-A-99/32045
- WO-A1-99/26540
- DE-A1- 19 725 197
- JP-A- 2003 088 537
- US-A- 4 837 732
- US-B1- 6 319 006
- SEIPEL S ET AL: "ORAL IMPLANT TREATMENT PLANNING IN A VIRTUAL REALITY ENVIRONMENT" 1 January 1998 (1998-01-01), COMPUTER METHODS AND PROGRAMS IN BIOMEDICINE, ELSEVIER, AMSTERDAM, NL, PAGE(S) 95 - 103 , XP000911203 ISSN: 0169-2607 * the whole document *

## Description

The present invention relates to an artificial tooth root implantation position determining instrument for determining, on the basis of mechanical analysis, the implantation position of an artificial tooth root for supporting false teeth that supplement lost portions of dentition.

### BACKGROUND ART

In cases where teeth are lost, a therapeutic technique is used in which an artificial tooth root cavity of the desired shape is formed by planing the jaw bone beneath the mucous membrane in the lost portion of the dentition, an artificial tooth root (implant) is implanted in the artificial tooth root cavity thus formed, and the abovementioned lost portion [of the dentition] is supplemented by supporting an artificial tooth on the implanted artificial tooth root.

Since an artificial tooth root is implanted beneath the mucous membrane, and the end [of this root] that protrudes from the mucous membrane is covered by an artificial tooth, the spring required in conventional tooth insertion therapy is eliminated so that the appearance is favorable. Furthermore, an artificial tooth supported by an artificial tooth root is not only similar to a natural tooth in terms of the repair procedure, but is also superior in terms of stability.

In cases where an artificial tooth root of the type described above is implanted, the dental physician takes tooth molds in the state in which teeth are lost in respective patients, and manufactures dentition pattern molds on the basis of these tooth molds. The manufactured dentition pattern molded is handed over to a dental technician, and the dental technician predicts the shape of the dental crown that supplements the lost portion [of the dentition] from the shapes of the respective remaining teeth indicated by this dentition pattern mold; the dental technician then prepares a wax artificial tooth called a "wax up" that conforms to this lost portion. Next, the dental physician prepares a diagnostic stent by replacing the wax artificial tooth based on the wax up with an acrylic resin, opens a hole in the center of the tooth portion of the diagnostic stent with a drill, and engages a roentgen-impermeable stopping inside this hole.

Furthermore, in a state in which such a diagnostic stent is mounted in the oral cavity of the patient, the dental physician performs CT (computed tomography) imaging, predicts the length, thickness and implantation position of the artificial tooth root that is to be implanted on the basis of the acquired CT image and dentition pattern mold, and selects an artificial tooth root corresponding to the predicted length, thickness and the like from structural bodies (artificial tooth roots) that have already been manufactured from (for example) titanium.

Then, the dental physician modifies the diagnostic stent to a surgical treatment stent, and, utilizing this surgical treatment stent, the dental physician bores an artificial tooth root cavity by means of a drill in order to implant the selected artificial tooth root, and implants the artificial tooth root in the appropriate position by engaging the titanium artificial tooth root in the artificial tooth root cavity thus formed. Furthermore, the missing portion [of the dentition] can be supplemented by means of an artificial tooth that matches the dentition of the patient by covering the end portion of the implanted artificial tooth root that protrudes from the mucous membrane with the [abovementioned] artificial tooth having the shape formed by the dental technician.

Here, the dental physician predicts the position in which the artificial tooth root is to be implanted from the direction of the opaque stopping of the diagnostic stent and the cross-sectional image of the jaw bone on the CT film; however, in cases where the direction of the opaque stopping of the diagnostic stent is inappropriate, the surgical treatment stent must be repaired using the intuition and experience of the dental physician. Furthermore, since the CT film is a two-dimensional image, it is difficult to obtain a three dimensional grasp of the tooth and jaw bone.

For example, there is a thick nervous system called the lower jaw channel in the lower jaw bone, and there is a cavity called the upper jaw cavity in the upper jaw; furthermore, tooth roots of the adjacent remaining teeth are present in the vicinity of the lost portion where the artificial tooth root is to be implanted, and it is extremely difficult to bore an artificial tooth root cavity while avoiding such nervous systems, cavities, tooth roots of remaining teeth and the like.

Accordingly, software that inputs CT data into a computer and performs a simulation has been developed, such as SimPlant manufactured by Materialize Co., which forms two-dimensional data into three-dimensional data in a computer, and allows a simulation to be performed, and the system disclosed in the specification of European Patent Application Patent Disclosure No. EP1036543A1.

However, the abovementioned software only performs simulations; in order to further utilize the results of the simulation thus performed, auxiliary members with guide holes that aid in the hole boring treatment of the artificial tooth root cavity have been proposed, such as SurgiCase manufactured by Materialize Co., or the members disclosed in Japanese Patent Application Laid-Open No. 2001-170080, Japanese Patent Publication No. 2001-523509, Japanese Patent Application Laid-Open No. 2003-88537 and the like.

By using such an auxiliary member in the artificial tooth root cavity boring procedure, the dental physician can perform an artificial tooth root boring treatment in accordance with the guidance of guide holes provided on the basis of CT images and the like for respective patients, so that even a dental physician with little experience in boring procedures can realize a procedure that ensures a certain degree of stability and security.

However, for example, in cases where the patient has an existing metal cap on a tooth that has already been treated, the X-ray beam during CT imaging is reflected by this cap, so that artifacts are generated in the CT data that is acquired, and the image quality of the dentition areas in particular deteriorates so that this dentition is difficult to see; as a result, it is difficult for the dental physician to predict an appropriate artificial tooth root implantation position.

Furthermore, in the artificial tooth root cavity boring procedure using the abovementioned method, although it is possible to bore an artificial tooth root cavity in an anatomically safe position, absolutely no consideration is given to mechanical evaluation factors generated in the nearby jaw bone by the stress received from the opposing tooth during masticating movement and the like.

Furthermore, it is relatively difficult to securely stabilize the auxiliary member that aids in the abovementioned artificial tooth root cavity boring procedure inside the oral cavity; in addition, even in cases where a boring procedure is performed utilizing a stabilized auxiliary member, since the boring procedure is performed by the dental physician, it is difficult to securely prevent human error.

Moreover, in the abovementioned method, a dental technician must form the final artificial tooth shape on the basis of a dentition pattern mold of the patient prepared by the dental physician; accordingly, skill, time and cost are required for the preparation of the dentition pattern mold and the formation of the artificial tooth by the dental technician.

XP-000911203 discloses an oral implant treatment planning in a virtual reality environment.

### DISCLOSURE OF THE INVENTION

The present invention is disclosed in claim 1 and was devised in light of the above facts; it is an object of the present invention to provide an artificial tooth root implantation position determining instrument and artificial tooth root implantation position determining method which create combined data without image artifacts by combining three-dimensional dentition data and jaw bone CT data acquired from respective patients, obtain a three-dimensional grasp of the shapes of the jaw bone beneath the mucous membrane and tooth roots of respective teeth in respective patients on the basis of the created three-dimensional data, and determine artificial tooth root implantation positions that sufficiently avoid the lower jaw channel, upper jaw cavity and adjacent tooth roots, a computer program for realizing this instrument by means of a computer, and a recording medium for recording this computer program.

Furthermore, it is another object of the present invention to provide an artificial tooth root implantation position determining instrument which determine implantation positions that reduce the load on the jaw bone caused by masticating movement, this being accomplished by determining implantation positions with mechanical evaluation factors generated in the jaw bone in the vicinity of the position where the artificial tooth root is to be implanted being taken into account.

Furthermore, it is another object outside the claimed invention to provide a guide member manufacturing device which aids in the artificial tooth root cavity boring procedure performed by the dental physician by utilizing various types of data created by the abovementioned artificial tooth root implantation position determining instrument, and an artificial tooth manufacturing device which manufactures an artificial tooth that supplements the lost portion of the dentition of the patient.

Furthermore, in order to further reduce human error in the boring procedure using a guide member, it is another object outside the claimed invention to provide a sensor which detects the boring direction of the boring device that bores the artificial tooth root cavity, and provides notification in cases where the detected boring direction is inappropriate, and a drill which has marks based on the boring distance in accordance with the implantation position determined by the abovementioned artificial tooth root implantation position determining instrument, and which is mounted in the boring device during use.

Furthermore, it is still another object outside the claimed invention to provide an artificial tooth root implantation position determining instrument which can cause various types of processing to be performed by a computer by acquiring and processing various types of data as digital data, thus simplifying and improving the accuracy of the work performed by the dental physician and dental technician.

The artificial tooth root implantation position determining instrument of the present invention is an artificial tooth root implantation position determining instrument for determining the implantation position of an artificial tooth root which supports an artificial tooth that supplements the lost portion of dentition, comprising dentition data acquisition means for acquiring three-dimensional data relating to the abovementioned dentition, jaw bone data acquisition means for acquiring three-dimensional data relating to the jaw bone connected to the abovementioned dentition, combining means for combining the three-dimensional data relating to dentition acquired by the abovementioned dentition data acquisition means and the three-dimensional data relating to the jaw bone acquired by the abovementioned jaw bone data acquisition means, artificial tooth data creating means for creating artificial tooth data indicating an artificial tooth that supplements the lost portion of the abovementioned dentition indicated by the combined data for dentition and jaw bone created by the abovementioned combining means, candidate receiving means for receiving a plurality of candidates for the implantation position of the abovementioned artificial tooth root on the basis of combined data to which artificial tooth data created by the abovementioned artificial tooth data creating means has been added, and determining means for determining one implantation position from the candidates received by the abovementioned candidate receiving means.

In cases where the present invention is used, combined data that is free of image artifacts can be created by combining three-dimensional data relating to dentition and three-dimensional data relating to the jaw bone that is connected to this dentition, so that the image quality can be improved. Furthermore, the shapes of the jaw bone beneath the mucous membrane and tooth roots of respective teeth can be grasped in three-dimensional terms for respective patients on the basis of this combined data, so that implantation positions for artificial tooth roots that sufficiently avoid the lower jaw channel, upper jaw cavity and adjacent tooth roots can be determined.

Furthermore, artificial tooth data indicating artificial teeth that supplement lost portions of the dentition can be created from the combined data thus obtained, and in cases where candidates for artificial tooth root implantation positions that take into account anatomical safety and mechanical stability are received on the basis of combined data to which such created artificial tooth data has been added, implantation positions with greater anatomical safety and higher mechanical stability can be determined. Moreover, if the respective types of data are acquired as digital data as described above, various types of processing used to determine the implantation positions of artificial tooth roots can be performed by a computer in an anatomically accurate manner using three-dimension steric bodies and two-dimensional planar images; furthermore, by subjecting various types of data to a file conversion, it is possible to achieve an even more accurate determination of implantation positions that allow an expectation of predictability by means of mechanical analysis. Moreover, the reutilization of various types of data is possible, so that even in cases where teeth present at the time of acquisition of respective data are subsequently lost, artificial teeth can be formed on the basis of three-dimensional data relating to dentition and the jaw bone acquired beforehand, so that artificial teeth that conform appropriately to remaining teeth and artificial tooth roots for supporting these artificial teeth can be implanted.

The artificial tooth root implantation position determining instrument of the present invention is characterized in that this instrument comprises mechanical evaluation factor calculating means for calculating the mechanical evaluation factors generated in the respective vicinities by a preset occlusion force for the implantation positions respectively indicated by each of the candidates received by the abovementioned candidate receiving means, and the abovementioned determining means are constructed so that the implantation position in which the mechanical evaluation factor calculated by the abovementioned mechanical evaluation factor calculating means shows a minimum value is determined.

In cases where this invention is used, principal stresses, equivalent stresses, strain energy and the like which are mechanical evaluation factors (mechanical parameters) generated in the jaw bone in respective vicinities by specified occlusion forces can be calculated for the plurality of artificial tooth root implantation positions received as candidates, and the implantation position whe4re4 the calculated mechanical evaluation factors show minimum values can be determined, so that an implantation position in which the mechanical evaluation factors generated in the jaw bone in the vicinity of the implanted artificial tooth root are further reduced can be determined.

The artificial tooth root implantation position determining instrument of the present invention is characterized in that the abovementioned three-dimensional data relating to the jaw bone includes hardness information for the abovementioned jaw bone, and the abovementioned mechanical evaluation factor calculating means are constructed so that the abovementioned mechanical evaluation factors are calculated on the basis of the abovementioned hardness information.

In cases where this invention is used, mechanical evaluation factors generated in the jaw bone in the vicinity of respective implantation sites received as candidates can be calculated on the basis of hardness information for respective parts of the jaw bone included in the three-dimensional data relating to the jaw bone that is acquired from the patient, so that mechanical evaluation factors in the nearby jaw bone can be calculated with good precision, thus allowing reliable determination of implantation sites in which the mechanical evaluation factors exerted on nearby areas by the implanted artificial tooth root are further reduced.

The artificial tooth root implantation position determining instrument of the present invention is an artificial tooth root implantation position determining instrument for determining the implantation position of an artificial tooth root which supports an artificial tooth that supplements the lost portion of dentition, comprising dentition data acquisition means for acquiring three-dimensional data relating to the abovementioned dentition, jaw bone data acquisition means for acquiring three-dimensional data relating to the jaw bone connected to the abovementioned dentition, combining means for combining the three-dimensional data relating to dentition acquired by the abovementioned dentition data acquisition means and the three-dimensional data relating to the jaw bone acquired by the abovementioned jaw bone data acquisition means, artificial tooth data creating means for creating artificial tooth data indicating an artificial tooth that supplements the lost portion of the abovementioned dentition indicated by the combined data for dentition and jaw bone created by the abovementioned combining means, occlusion information creating means for creating occlusion information in the artificial tooth indicated by the artificial tooth data created by the abovementioned artificial tooth data creating means on the basis of the abovementioned combined data, and calculating means for calculating the implantation position of the artificial tooth root that supports the abovementioned artificial tooth on the basis of the occlusion information created by the abovementioned occlusion information creating means.

In cases where this invention is used, artificial tooth data indicating an artificial tooth that supplements the lost portion of the dentition is created from combined data obtained by combining three-dimensional data relating to the dentition and three-dimensional data relating to the jaw bone that is connected to this dentition. Furthermore, occlusion information indicating the occlusion state of the remaining tooth or artificial tooth in use that opposes the artificial tooth indicated by the created artificial tooth data is created on the basis of the combined data thus obtained, and the artificial tooth root implantation position is calculated on the basis of the created occlusion information. As a result, an artificial tooth root implantation position that is suited to the state of occlusion with the opposing remaining tooth or artificial tooth in use can be calculated.

The artificial tooth root implantation position determining instrument of the present invention is an artificial tooth root implantation position determining instrument for determining the implantation position of an artificial tooth root which supports an artificial tooth that supplements the lost portion of dentition, comprising dentition data acquisition means for acquiring three-dimensional data relating to the abovementioned dentition, jaw bone data acquisition means for acquiring three-dimensional data relating to the jaw bone connected to the abovementioned dentition, mastication information acquisition means for acquiring mastication information the abovementioned dentition, combining means for combining the three-dimensional data relating to dentition acquired by the abovementioned dentition data acquisition means and the three-dimensional data relating to the jaw bone acquired by the abovementioned jaw bone data acquisition means, artificial tooth data creating means for creating artificial tooth data indicating an artificial tooth that supplements the lost portion of the abovementioned dentition indicated by the combined data for dentition and jaw bone created by the abovementioned combining means, occlusion information creating means for creating occlusion information in the artificial tooth indicated by the artificial tooth data created by the abovementioned artificial tooth data creating means on the basis of the abovementioned combined data and the mastication information acquired by the abovementioned mastication information acquisition means, and calculating means for calculating the implantation position of the artificial tooth root that supports the abovementioned artificial tooth on the basis of the occlusion information created by the abovementioned occlusion information creating means.

In cases where this invention is used, mastication information in the dentition is acquired along with three-dimensional data relating to the dentition and three-dimensional data relating to the jaw bone. Furthermore, artificial tooth data indicating an artificial tooth that supplements the lost portion of the dentition is created from combined data obtained by combining the three-dimensional data relating to the dentition and the three-dimensional data relating to the jawbone connected to this dentition. Furthermore, occlusion information indicating the state of occlusion of the tooth opposing the artificial tooth indicated by the created artificial tooth data is created from mastication information acquired beforehand in the dentition indicated by the combined data thus obtained, and the artificial tooth root implantation position is calculated on the basis of the created occlusion information. As a result, an artificial tooth root implantation position that is suited to the state of occlusion with the remaining tooth or artificial tooth in use that is opposite during the mastication operation of the patient can be calculated.

The artificial tooth root implantation position determining instrument of the present invention comprises candidate receiving means for receiving a plurality of candidates for the implantation position of the abovementioned artificial tooth root on the basis of combined data to which the artificial tooth data created by the abovementioned artificial tooth data creating means has been added, and mechanical evaluation factor calculating means for calculating the mechanical evaluation factors generated in the respective vicinities by an occlusion force indicated by occlusion information created by the occlusion information creating means for the implantation positions respectively indicated by each of the candidates received by the abovementioned candidate receiving means, and the abovementioned calculating means are constructed so that the implantation position in which the mechanical evaluation factor calculated by the abovementioned mechanical evaluation factor calculating means shows a minimum value is determined.

In cases where this invention is used, the mechanical evaluation factors generated in the nearby jaw bone by the occlusion force indicated by the occlusion information for the artificial tooth created in the dentition indicated by the combined data is calculated for the plurality of artificial tooth root implantation positions received as candidates, and a mechanically stable implantation position in which the mechanical evaluation factors generated in the nearby jaw bone by the implanted artificial tooth root is further reduced is determined by determining the implantation position in which the calculated mechanical evaluation factors show minimum values.

The artificial tooth root implantation position determining instrument of the present invention is characterized in that the abovementioned artificial tooth data creating means are constructed so as to create the abovementioned artificial tooth data on the basis of the remaining teeth in the dentition indicated by the abovementioned combined data.

In cases where this invention is used, the artificial tooth data indicating the artificial tooth that supplements the lost portion of the dentition indicated by the combined data is created on the basis of the remaining teeth in this dentition, so that artificial tooth data suited to the remaining teeth in the dentition can be calculated, thus making it possible to calculate an artificial tooth root implantation position supporting the artificial tooth from teeth and artificial teeth conforming to the jaws of respective patients.

The artificial tooth root implantation position determining instrument of the present invention is characterized in that the abovementioned artificial tooth data creating means are constructed so that in cases where there are no remaining teeth in the dentition indicated by the abovementioned combined data, the abovementioned artificial tooth data is created on the basis of artificial teeth in use.

In cases where this invention is used, if there are no remaining teeth in the dentition indicated by the combined data, artificial tooth data is created on the basis of artificial teeth that are in use in this dentition, so that even in cases where there are no remaining teeth, artificial tooth data that conforms to the artificial teeth in use in the dentition can be calculated, and implantation positions for artificial tooth roots that support artificial teeth can be calculated from artificial teeth that are in conformity with the teeth and jaws of respective patients.

The artificial tooth root implantation position determining method is an artificial tooth root implantation position determining method for determining the implantation position of an artificial tooth root which supports an artificial tooth that supplements the lost portion of dentition using a calculating device comprising a receiving part that receives data from the outside, a storage part and a calculating part, wherein three-dimensional data relating to the abovementioned dentition and three-dimensional data relating to the jaw bone connected to the abovementioned dentition are received by the receiving part, the abovementioned received three-dimensional data relating to the dentition and three-dimensional data relating to the jaw bone are stored in the storage part, the abovementioned stored three-dimensional data relating to the dentition and three-dimensional data relating to the jaw bone are combined by the abovementioned calculating part, artificial tooth data indicating an artificial tooth that supplements the lost portion of the abovementioned dentition indicated by the created dentition and jaw bone combined data is created by the calculating part, occlusion information in the artificial tooth indicated by the created artificial tooth data is created by the calculating part on the basis of the abovementioned combined data, and the implantation position of the artificial tooth root that supports the abovementioned artificial tooth is calculated by the abovementioned calculating part on the basis of the created occlusion information.

In cases where this invention is used, three-dimensional data relating to the dentition and three-dimensional data relating to the jaw bone connected to this dentition received from the outside are stored in a storage part, this three-dimensional data relating to dentition and three-dimensional data relating to the jaw bone are combined by a calculating part, and artificial tooth data indicating an artificial tooth that supplements the lost portion of the abovementioned dentition is created from the combined data thus obtained. Furthermore, the implantation position of the artificial tooth root that supports the abovementioned artificial tooth is calculated on the basis of occlusion information in the artificial tooth indicated by the artificial tooth data created on the basis of the combined data, so that appropriate implantation positions can be selected from received candidates with a three-dimensional grasp of the shapes of the jaw bone beneath the mucous membrane and tooth roots of the respective teeth, thus making it possible to determine artificial tooth root implantation positions that conform to the occlusion state of the opposing remaining teeth or artificial teeth in use, and that further reduce the mechanical evaluation factors generated in the nearby jaw bone.

The guide member manufacturing device outside the claimed invention is a guide member manufacturing device which manufactures a guide member having a guide hole that communicates with the artificial tooth root cavity in which an artificial tooth root supporting an artificial tooth that supplements the lost portion of dentition is implanted, comprising forming means for forming a member that covers the artificial tooth indicated by artificial tooth data, and the vicinity of the abovementioned artificial tooth, on the basis of combined data to which artificial tooth data created by the artificial tooth root implantation position determining instrument according to any one of claims 1 through 8 has been added, and hole boring means for boring the abovementioned guide hole that communicates with the implantation position determined by the abovementioned artificial tooth root implantation position determining instrument in the member formed by the abovementioned forming means.

In cases where this invention is used, the artificial tooth data and implantation position created by the abovementioned artificial tooth root implantation position determining instrument are acquired, a member with a shape that covers the artificial tooth indicated by the abovementioned artificial tooth data and the vicinity of this artificial tooth is formed on the basis of combined data to which the abovementioned artificial tooth data has been added; furthermore, as a result of a guide hole that communicates with the acquired implantation position being bored in the formed member, this guide hole guides the tip end portion of the hole boring device such as a drill or the like during the boring of the artificial tooth root cavity, so that this artificial tooth root cavity can be bored with good precision in the appropriately calculated implantation position.

The sensor outside the claimed invention is a sensor which is mounted in a hole boring device that bores an artificial tooth root cavity in which an artificial tooth root is implanted, and which detects the hole boring direction of the abovementioned hole boring device, comprising detection means for detecting the hole boring direction of the abovementioned hole boring device, judgment means for judging whether or not the hole boring direction detected by the abovementioned detection means is the boring direction of the guide hole possessed by the guide member manufactured by the guide member manufacturing device of the present invention, and notification means for making notification that the boring direction of the abovementioned boring device is erroneous in cases where it is judged by the abovementioned judgment means that the boring direction is not the boring direction of the abovementioned guide hole.

In cases where this invention is used, for example, since [a sensor] is mounted on the hole boring device such as a drill device or the like, and since the hole boring direction of the boring operation of this hole boring device is detected, and notification is made that the hole boring direction of the hole boring device is erroneous in cases where the detected hole boring direction is not the boring direction of the guide hole of the guide member manufactured by the abovementioned guide member manufacturing device, even a dental physician who has little experience in artificial tooth root cavity boring procedures can bore an artificial tooth root cavity with good precision along the guide hole of the guide member.

The drill outside the claimed invention is a drill mounted in a boring device, wherein this drill has marks spaced at a distance from the tip end portion based on the implantation position determined by the artificial tooth root implantation position determining instrument of the present invention.

In cases where this invention is used, a hole boring treatment is performed while referring to marks in positions spaced at a distance from the tip end portion based on the implantation position determined by the abovementioned artificial tooth root implantation position determining instrument; accordingly, even a dental physician with little experience in hole boring procedures can bore a hole with a hole boring distance in an implantation position determined with good precision, so that an anatomically safe hole boring procedure is possible. Furthermore, in cases where this is utilized together with the abovementioned guide member, a hole boring treatment can be performed while maintaining an appropriate hold boring angle and hole boring distance.

The artificial tooth manufacturing device outside the claimed invention is an artificial tooth manufacturing device for manufacturing an artificial tooth that supplements the lost portion of dentition, comprising planing means for planing an artificial tooth which has an occlusion face indicated by occlusion information and a shape indicated by artificial tooth data on the basis of the artificial tooth data and occlusion information created by the artificial tooth root implantation position determining instrument of the present invention.

In cases where this invention is used, artificial tooth data and occlusion information created by the abovementioned artificial tooth root implantation position determining instrument are acquired, and an artificial tooth with an occlusion face indicated by this occlusion information and a shape indicated by the artificial tooth data is formed by planing, so that the artificial tooth data and occlusion information obtained can be utilized in the calculation of an appropriate artificial tooth root implantation position, thus making it possible to manufacture with good precision an artificial tooth that conforms to the occlusion operation and mastication movement.

The computer program is a computer program for causing a computer to determine the implantation position of an artificial tooth root which supports an artificial tooth that supports the lost portion of dentition, wherein this computer program executes a routine that causes the computer to combine acquired three-dimensional data relating to dentition and three-dimensional data relating to the jaw bone connected to the abovementioned dentition, a routine that causes the computer to create artificial tooth data indicating an artificial tooth that supplements the lost portion of the abovementioned dentition indicated by the created combined data relating to the dentition and jaw bone, a routine that causes the computer to create occlusion information in the artificial tooth indicated by the created artificial tooth data on the basis of the abovementioned combined data, and a routine that causes the computer to calculate the implantation position of the artificial tooth root that supports the abovementioned artificial tooth on the basis of the created occlusion information.

In cases where this invention is used, the abovementioned artificial tooth root implantation position determining instrument can be realized as a CAD system using a computer by reading [the program] into a computer and causing the computer to execute the [abovementioned] routines, so that artificial tooth root implantation positions corresponding to the shapes of the dentition and jaw bones of individual patients can be determined by computer processing.

The recording medium is a computer-readable recording medium on which a computer program that is used to cause a computer to determine the implantation position of an artificial tooth root which supports an artificial tooth that supplements the lost portion of dentition is recorded, wherein this recording medium records a computer program that executes a routine that causes the computer to combine acquired three-dimensional data relating to dentition and three-dimensional data relating to the jaw bone connected to the abovementioned dentition, a routine that causes the computer to create artificial tooth data indicating an artificial tooth that supplements the lost portion of the abovementioned dentition indicated by the created combined data relating to the dentition and jaw bone, a routine that causes the computer to create occlusion information in the artificial tooth indicated by the created artificial tooth data on the basis of the abovementioned combined data, and a routine that causes the computer to calculate the implantation position of the artificial tooth root that supports the abovementioned artificial tooth on the basis of the created occlusion information.

In cases where this invention is used, a recorded computer program can be read into a computer and the computer can be caused to execute routines so that the abovementioned artificial tooth root implantation position determining instrument can be realized by means of an arbitrary computer.

### BRIEF DESCRIPTION OF THE DRAWINGS

Fig. 1 is a block diagram showing an example of the construction of an artificial tooth root implantation position determining instrument constituting a first embodiment of the present invention;
Fig. 2 is an explanatory diagram of dentition data;
Fig. 3 is an explanatory diagram of jaw bone data;
Fig. 4 is an explanatory diagram of combined data;
Fig. 5 is a flow chart showing the artificial tooth root implantation determining processing routine performed by a computer;
Fig. 6 is a flow chart showing the dental crown data creation processing routine performed by a computer;
Fig. 7 is a flow chart showing the dental crown data creation processing routine performed by a computer;
Fig. 8 is a flow chart showing the occlusion data creation processing routine performed by a computer;
Fig. 9 is a flow chart showing the artificial tooth root implantation position calculation processing routine performed by a computer;
Fig. 10 is a block diagram showing an example of the construction of the guide member manufacturing device outside the claimed invention;
Fig. 11 is a flow chart showing the guide member manufacturing processing routine performed by the guide member manufacturing device outside the claimed invention;
Fig. 12 is a model diagram showing one example of the guide member;
Fig. 13 is a block diagram showing an example of the construction of the sensor outside the claimed invention;
Fig. 14 is a flow chart showing the artificial tooth root cavity boring processing routine performed by a hole boring device mounting the detection part outside the claimed invention;
Fig. 15 is a block diagram showing an example of the construction of the artificial tooth manufacturing device outside the claimed invention;
Fig. 16 is a flow chart showing the dental crown manufacturing processing routine performed by the artificial tooth manufacturing device outside the claimed invention;
Fig. 17 is a block diagram showing an example of the construction of an artificial tooth root implantation position determining instrument constituting a second embodiment;
Fig. 18 is a flow chart showing the artificial tooth root implantation position determining processing routine performed by a computer; and
Fig. 19 is an explanatory diagram of the artificial tooth root implantation position determination processing performed by a computer in the second embodiment.

### BEST MODE FOR CARRYINGN OUT THE INVENTION

Embodiments of the present invention will be concretely described below with reference to the attached figures.

### (First Embodiment)

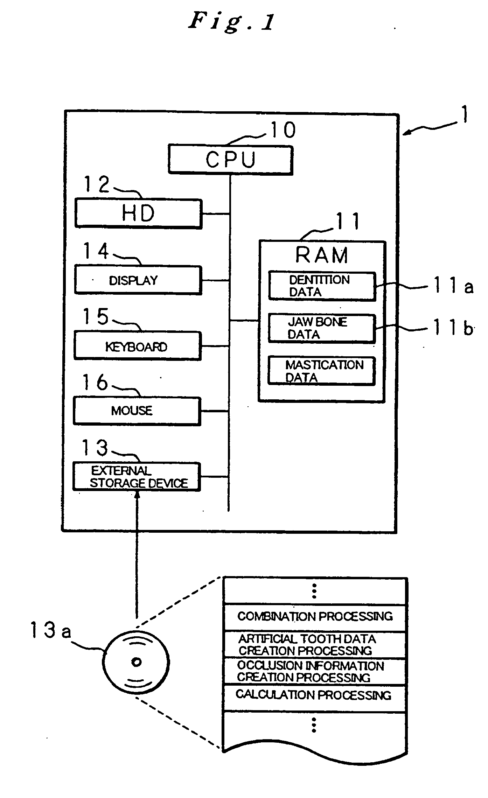

Fig. 1 is a block diagram showing one example of th construction of an artificial tooth root implantation position determining instrument constituting a first embodiment of the present invention. Fig. 1 shows a computer constituting the artificial tooth root implantation position determining instrument of the present invention.

The computer 1 comprises a CPU 10, RAM 11, hard disk (hereafter referred to as an "HD") 12, external storage device 13, display 14, keyboard 15, mouse 16 and the like.

The CPU 10 is connected to the various abovementioned hardware parts of the computer 1 via a bus; this CPU 10 controls these hardware parts, and successively executes computer programs stored in the HD 12. The HD 12 stores various computer programs that are necessary for the operation of the artificial tooth root implantation position determining instrument of the present invention.

The RAM 11 is constructed from an SRAM, DRAM, flash memory or the like, and stores temporary data that is generated during the execution of computer programs by the CPU 10. Furthermore, in cases where a flash memory is used for the RAM 11, there is no loss of the stored contents even in cases where the power supply is cut off due to a power failure, movement of the computer I or the like.

The external storage device 13 is constructed from a CD-ROM drive, flexible disk drive or the like, and the combination processing program, artificial tooth data creation processing program, occlusion information processing program and calculation processing program which are computer programs of the present invention are read out from the portable storage medium 13a such as a CD-ROM, flexible disk drive or the like in which the computer programs of the present invention are recorded, and are stored in the HD 12.

Accordingly, the CPU 10 can also appropriately read out (to the RAM 11) and execute computer programs that are acquired via the external storage device 13 and stored in the HD 12 in the same manner as computer programs stored beforehand in the HD 12.

In cases where the computer 1 constructed as described above operates as an artificial tooth root implantation position determining instrument, this computer 1 respectively acquires dentition data 11a that expresses the dentition of respective patients as three-dimensional data, jaw bone data 11 b that expresses the jaw bone connected to the dentition as three-dimensional data, and mastication data that indicates operating information during mastication (mastication information), from the outside. Accordingly, the CPU 10 operates as dentition data acquisition means for acquiring dentition data 11a, jaw bone data acquisition means for acquiring jaw bone data 11b, and mastication information acquisition means for acquiring mastication data.

Here, the dental physician acquires these various types of data beforehand using various types of devices that will be described below; then, when artificial tooth root implantation position determination processing is executed, these various types of data are input into the computer 1. The computer 1 stores the various types of acquired data in the HD 12, and reads this data out into the RAM 11 and performs processing as required.

Furthermore, a construction may be used in which the computer 1 and various devices are connected via cables, and various types of data are input into the computer 1 from these respective devices via these cables, or a construction may be used in which various types of data that are temporarily stored in a storage medium such as a CD-ROM, flexible disk or the like from respective devices are input into the computer 1 via the external storage device 13.

The acquisition processing of the dentition data 11a, jaw bone data 11b and mastication data in the present embodiment will be described below.

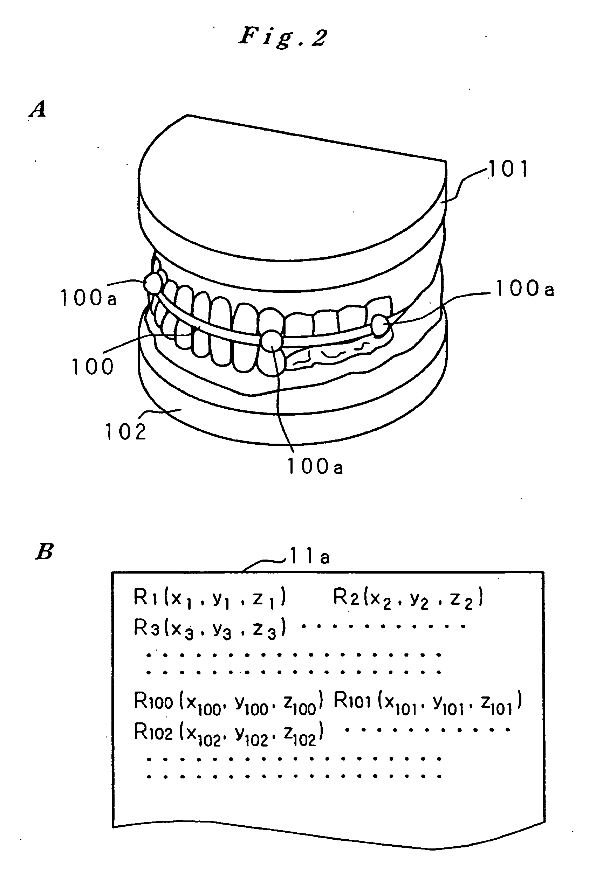

First, in cases where dentition data 11a is acquired, for example, a device such as the Surfracer manufactured by Unison K.K., Fastscan manufactured by Nissho Electronics K.K., LPX-250 manufactured by Roland DG K.K. or the like (called a non-contact type three-dimensional digitizer, non-contact type three-dimensional configuration reader, and non-contact type laser scanner or the like) is used. As is shown in Fig. 2 (a), these devices acquire dentition data 11a which is three-dimensional data from dentition pattern molds 101 and 102 prepared on the basis of tooth models acquired from the patients. Furthermore, in lost portions of the dentition, the surface of the mucous membrane (jaw line) is acquired as three-dimensional data.

Here, when dentition data 11a is acquired, in the case of patients with relatively large numbers of remaining teeth, as is shown in Fig. 2 (a), a horseshoe shaped compound 100 such as Aruwax (registered trademark) manufactured by Aru K.K. is softened between the dentition pattern molds 101 and 102 prepared on the basis of the upper and lower jaws, and occlusion is effected. Furthermore, the substance used for this occlusion is not limited to this compound 100; a silicon material or wax material may also be used.

Furthermore, while this compound 100 is in a softened state, ceramic balls 100a, 100a ... with a diameter of approximately 7 mm are pressed against the outside of the compound 100 in specified locations (three locations in the figures), and dentition data 11a is acquired in a state in which the ceramic balls 100a, 100a ... are fastened in place by means of a bonding agent or the like. Furthermore, the members that are pressed against the compound 100 are not limited to these ceramic balls 100a, and the size is likewise not limited to 7 mm, as long as these members are manufactured with good precision from a material that does not generate metal artifacts in the CT images.

On the other hand, in the case of patients with many missing teeth, dentition pattern molds are prepared in a state in which artificial teeth in use (false teeth) that are used by the patient mounted in place, and dentition data 11a is acquired by the same method as that described above.

As is shown in Fig. 2 (b), for example, such dentition data 11a is acquired with the initially detected pint R1 in the dentition used as a reference, with the outlines of the dentition pattern molds 101 and 102, compound 100 and ceramic balls 100a, 100a ... detected while being shifted by a specified distance in the x, y and z directions in a three-dimensional space, and with the surface shapes acquired as point data.

Next, in cases where jaw bone data 11b is acquired, a CT imaging device is used which acquires tomographic images of the internal organs of the human body while performing a scan with an X-ray beam. The CT imaging device acquires a plurality of two-dimensional CT images by imaging the jaw bone of the patient, and acquires three-dimensional jaw bone data 11b by synthesizing these images into three-dimensional data.

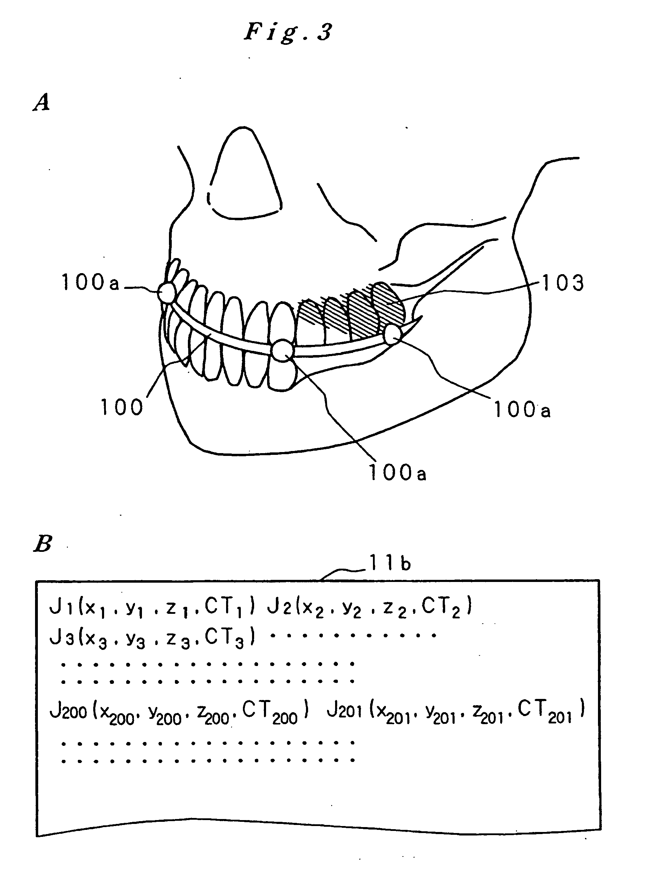

Here, in the acquisition of CT images, as in the case of the acquisition of dentition data 11a, when the patient has a relatively large number of remaining teeth, a compound 100 with ceramic balls 100a, 100a ... attached in appropriate places is occluded between the upper and lower jaws as shown in Fig. 3 (a). Furthermore, in the case of patients with relatively large numbers of missing teeth, artificial teeth that are in use are mounted, and CT images are acquired in a state in which the compound 100 is occluded by the dentition including these artificial teeth in use.

The CT images thus acquired are tomographic images of the jaw bone, and two-dimensional line data in the x and y directions indicating the respectively imaged jaw bone is created on the basis of these CT images. Furthermore, these CT images are continuously imaged in the z direction at a specified pitch, and three-dimensional line data can be created by connecting the line data created from the respective CT images in the z direction.

In the present embodiment, jaw bone data 11b in which the jaw bone is indicated by point data as shown in Fig. 3 (b) is acquired by extracting point data at appropriate intervals on the basis of the line data thus created. Furthermore, the processing that creates the jaw bone data 11b from the CT images can be performed by the computer 1, or can be performed by an external computer.

Furthermore, the CT images include CT values (hardness information) indicating the hardness of the object of imaging according to the transmissivity of the X-ray beam or the like, so that the jaw bone data 11b is constructed from point data that indicates the respective parts of the jaw bone, and CT values in the positions indicated by the respective point data.

Furthermore, bone portions can be imaged with good precision by appropriately varying the CT threshold value in the CT imaging device. Moreover, in cases where data imaged by a magnetic resonance device (MRI data) is added, not only bone portions, but also the shapes of soft tissues such as muscles, skin and the like can be grasped.

Finally, in cases where mastication data is acquired, for example, a mastication movement measuring device such as Sironaso manufactured by Tokyo Shika Sangyo K.K., Nasohexagraph manufactured by C.C. K.K. or the like is used. In cases where the patient is using artificial teeth, the patient is caused to chew a flexible substance in a state in which these artificial teeth are mounted in specified positions, and these devices acquire mastication data (mastication information) by measuring movement information of the dentition during this mastication.

In the computer 1 in which the dentition data 11a, jaw bone data 11b and mastication data acquired using respective devices as described above are stored in the RAM 11, the CPU 10 reads a combination processing program acquired via the external storage device 13 and stored in the HD 12 out into the RAM 11, and successively executes [the routines of] this program, thus operating as combining means that combine the dentition data 11a and jaw bone data 11b.

In concrete terms, the CPU 10 extracts the point data indicating the respectively included ceramic balls 100a, 100a ... from the dentition data 11a and jaw bone data 11b, and detects the center positions of the respective ceramic balls 100a, 100a .... Here, since the ceramic balls 100a, 100a ... in the dentition data 11a and jaw bone data 11b are the same, and since the respective sets of data are measured in actual dimensions, the CPU 10 combines the dentition data 11a and jaw bone data 11b using the center positions of the ceramic balls 100a, 100a ... in the respective sets of data as a reference, and stores the combined data in the RAM 11.

Furthermore, in regard to the occlusion face of the dentition, the dentition pattern molds of the upper jaw and lower jaw can be read out as the three-dimensional data from the direction of the occlusion face utilizing the same device used to acquire the dentition data 11a, and combined data including the occlusion face can be created by combining this information together with the dentition data 11a and jaw bone data 11b.

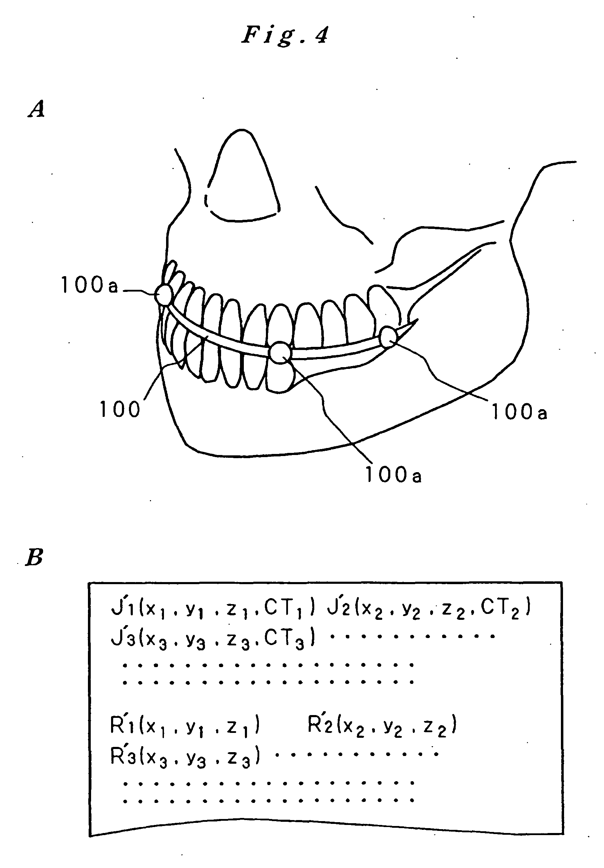

Fig. 4 (a) is a model diagram of the dentition and jaw bone in a case where the combined data created as described above is displayed as an image. As is shown in Fig. 4 (b), this combined data is constructed from the dentition data 11a shown in Fig. 2 (b) and the jaw bone data 11b shown in Fig. 3 (b).

Furthermore, as was described above, the dentition data 11a is acquired in a state in which the compound 100 to which the ceramic balls 100a, 100a ... are attached is occluded by the dentition pattern molds 101 and 102; furthermore, the jaw bone data 11b is created from CT images acquired in a state in which the compound 100 is occluded, so that data for the respective teeth and data for the jaw bone connected to the respective teeth can be appropriately combined using the ceramic balls 100a, 100a ... in the dentition data 11a and jaw bone data 11b as a reference.

Furthermore, in cases where the patient has metal caps on teeth that have already been treated, the X-ray beam used to acquire the CT images is reflected by such caps, so that artifacts are generated in the CT images; accordingly, data in places such as that indicated by 103 in Fig. 3 (a) cannot be appropriately acquired, and it is extremely difficult to acquire appropriate CT images of dentition information. Accordingly, appropriate combined data without image artifacts can be acquired by referring to existing literature (e. g., Orthod. Waves 57 (3): 189-194, 1998, Nishii et ai.), and combining dentition data 11a acquired from dentition pattern molds as described above and jaw bone data 11b created from CT images.

In the computer 1 in which combined data is created by combining dentition data 11a and jaw bone data 11b as described above, the CPU 10 reads out the artificial tooth data creation processing program acquired via the external storage device 13 and stored in the HD 12 into the RAM 11, and successively executes this program, thus operating as artificial tooth data creation means that create artificial tooth data indicating the artificial tooth that supplements the lost portion of the dentition indicated by the combined data that has been created. Here, the artificial tooth that supplements the lost portion of the dentition may be a dental crown supported by an artificial tooth root implanted in an artificial tooth root cavity formed by boring a hole in the jaw bone, a removable artificial tooth (insert) or the like. The computer 1 in the present embodiment creates dental crown data indicating a dental crown that is supported by an artificial tooth root.

In concrete terms, the computer 1 converts dental crown data reported as statistical data corresponding to race, facial silhouette shape and the like into a data base and stores this data in the HD 12. Dental crown data indicating dental crowns that are supported by artificial tooth roots can be created on the basis of dental crown data (hereafter referred to as "existing dental crown data") in this existing data base (hereafter referred to as a "DB"). Furthermore, the computer 1 can also create dental crown data on the basis of the remaining teeth possessed by the patient; the method by which dental crown data is created is determined by the choice of the dental physician.

More concretely, in cases where the dental physician chooses to create dental crown data on the basis of existing dental crown data, the CPU 10 detects the lost portion of the dentition indicated by the combined data, and reads out the existing dental crown data that is to repair the detected lost portion from the DB of the HD 12. Furthermore, if a tooth of the same name on the opposite side of the same jaw with respect to the detected lost portion remains without being repaired, dental crown data that agrees with the width of the remaining tooth of the same name is created by enlarging or reducing the existing dental crown data that is read out.

Furthermore, in cases where a tooth of the same name on the opposite side of the same jaw with respect to the lost portion does not remain, the CPU 10 calculates the width of the dental crown to repair the lost portion in accordance with a mean ratio obtained by statistical calculation on the basis of the widths of other teeth that remain in this same jaw, and creates dental crown data that agrees with the calculated width by enlarging or reducing the existing dental crown data that is read out from the DB.

Furthermore, statistical data accumulated for the respective human races (such as the Bolton Index for example) is used for the [abovementioned] mean ratio. The shapes of dental crowns created in accordance with such statistical data suitably conform to the shapes of remaining teeth and artificial teeth in use, and can be caused to show appropriate agreement with respective adjacent teeth.

Furthermore, in cases where there are no remaining teeth in the same jaw as the lost portion, the CPU 10 can similarly calculate the width of the dental crown that will repair the lost portion on the basis of the widths of the respective teeth that remain in the opposite jaw, and create dental crown data that agrees with the calculated width by enlarging or reducing the existing dental crown data that is read out from the DB. Furthermore, in cases where there are no remaining teeth in the opposite jaw, either, the CPU 10 can similarly create dental crown data on the basis of the widths of respective artificial teeth in false teeth being used by the patient.

Furthermore, in cases where the patient is not using any current artificial teeth, the CPU 10 measures the width of the ala nasi of the patient, calculates the width of the dental crown that will repair the lost portion on the basis of this ala nasi width, and creates dental crown data that agrees with the calculated width by enlarging or reducing the existing dental crown data that is read out from the DB. Furthermore, dental crown data can be created not only on the basis of the ala nasi width, but also on the basis of measured values of various parts of the patient's face, e. g., on the basis of the disposition positions of various parts with respect to the silhouette line of the nose or the like. Furthermore, the CPU 10 stores the created dental crown data in the RAM 11.

On the other hand, in cases where the dental physician chooses to create dental crown data on the basis of remaining teeth of the patient without using existing dental crown data, the CPU 10 detects the lost portion of the dentition indicated by the combined data, and if a tooth of the same name remains without being repaired on the opposite side of the same jaw with respect to the detected lost portion, dental crown data with a shape that is obtained by a mirror image transfer of this remaining tooth of the same name is created. Furthermore, since the method of dental crown data creation using such a mirror image transfer can be used only on teeth of the same name in the same jaw, it is necessary to perform dental crown data creation processing based on existing dental crown data accumulated in the DB as described above in the case of patients in which teeth of the same name in the same jaw with respect to the lost portion do not remain.

In the computer 1 that has created dental crown data to repair the lost portion of the dentition on the basis of combined data [combining] the dentition data 11a and jaw bone data 11b as described above, the CPU 10 reads out the occlusion information creation processing program that is acquired via the external storage device 13 and stored in the HD 12 into the RAM 11, and successively executes this program, thus operating as occlusion information creating means that create occlusion data (occlusion information) indicating the state of occlusion of the dental crown based on the created dental crown data with the opposing tooth.

Here, in the present embodiment, the acquisition of mastication data for respective patients is not considered to be an essential condition; in cases where mastication data is not acquired, the CPU 10 causes the occlusion of the respective teeth of the upper jaw and lower jaw in an appropriate occlusion state on the basis of the occlusion faces of the respective teeth indicated by the combined data, and in this state, creates occlusion data that indicates the occlusion state of the dental crown indicated by the dental crown data (added in an appropriate place in the combined data) with the opposing tooth, and stores this data in the RAM 11. Furthermore, the occlusion data indicates the stress and the like from the occlusion face of the dental crown in an appropriate occlusion state with the opposing tooth, and from the opposing tooth.

On the other and, in cases where mastication data is acquired, the CPU 10 causes virtual execution of mastication movement in the dentition indicated by the combined data by substituting the mastication data into the combined data, thus creating occlusion data indicating the occlusion state of the dental crown indicated by the dental crown data (added to the combined data) with the opposing tooth on the basis of the mastication movement peculiar to each patient, and stores this data in the RAM 11.

In the computer 1 that has created occlusion data for the dental crown data created in order to repair the lost portion as described above, the CPU 10 reads out the calculation processing program acquired via the external storage device 13 and stored in the HD 12 into the RAM 11, and successively executes this program, thus operating as calculation means which calculate the implantation position of the artificial tooth root that supports the dental crown indicated by the dental crown data on the basis of the created occlusion data. Furthermore, in the artificial tooth root implantation position determining instrument of the present invention, the implantation position is calculated by the finite element method with the occlusion data as the load condition in the dental crown data.

First, the CPU 10 combines the occlusion data created as described above with the combined data, so that the occlusion faces of the respective teeth in the combined data are formed into shapes that conform to the occlusion data, and stores this data in the RAM 11 for utilization in the manufacture of the artificial tooth as the final repair object.

The computer I of the present embodiment performs processing that determines the position where the artificial tooth root is to be implanted according to the optimal shape determination method on the basis of the abovementioned combined data and occlusion data, and processing that determines the optimal implantation position from candidates for the implantation position that are input by the dental physician. The processing that is performed is determined by the choice of the dental physician.

In cases where the appropriate implantation position is determined from candidates input by the dental physician, the CPU 10 operates as candidate receiving means that receive a plurality of sets of candidate data (two sets of candidate data in the present embodiment) for the artificial tooth root implantation position from the dental physician on the basis of the created combined data.

Furthermore, for the implantation positions indicated by the respective sets of candidate data received, the CPU 10 operates as mechanical evaluation factor calculation means that calculate the mechanical evaluation factors generated in the nearby jaw bone on the basis of the created occlusion data in cases where a specified stress is applied in the direction of the occlusion force received from the tooth opposing the dental crown indicated by the dental crown data. Here, the mechanical evaluation factors are mechanical parameters for evaluating the mechanical environment, and include (for example) the principal stress applied to the jaw bone, the equivalent stress, the amount of strain energy and the like.

Furthermore, the CPU 10 selects the artificial tooth root implantation position in which the calculated values of the mechanical evaluation factors are small, i. e., in which the mechanical evaluation factors generated in the nearby jaw bone are further reduced, and stores the candidate data indicating the selected implantation position in the RAM 11.

Here, as was described above, the jaw bone data 11b includes CT values that indicate hardness information for respective parts of the jaw bone, and the CPU 10 converts the CT values into material characteristics so that the finite element method can be used.

Furthermore, the stress applied to various parts of the jaw bone on the basis of the occlusion force received by the dental crown from the opposing tooth us multiplied by the material characteristics of the respective parts, and the mechanical evaluation factors generated in the jaw bone can be calculated with good precision by performing a finite element analysis in specified regions.

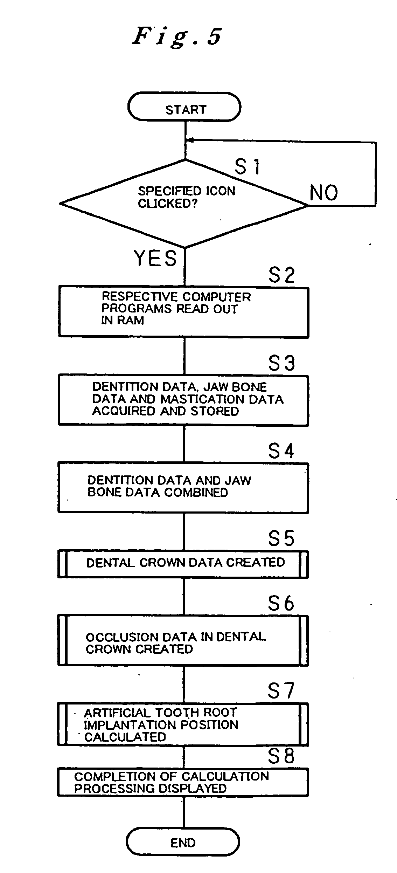

The processing that determines the artificial tooth root implantation position using a computer 1 with a construction that executes the various types of processing described above will be described below. Fig. 5 is a flow chart showing the artificial tooth root implantation position determining processing routine that is performed by the computer 1.

In cases where the computer 1 is caused to operate as an artificial tooth root implantation position determining instrument, the dental physician acquires dentition data 11a, jaw bone data 11b and mastication data for the subject patient beforehand by means of respective external devices, and causes the computer 1 to execute artificial tooth root implantation position determining processing by clicking on a specified icon disposed on the computer 1 by means of the mouse 16. Furthermore, mastication data need not be acquired.

In the computer 1, the CPU 10 judges whether or not the [abovementioned] specified icon has been clicked on (S 1). In cases where it is judged that the specified icon has not been clicked on (S1: NO), the computer waits until the specified icon has been clicked on. In cases where it is judged that the specified icon has been clicked on (S1: YES), the respective computer programs of the present invention stored in the HD 12 are read out into the RAM 11 (S2), and the program codes contained in the respective computer programs are successively executed.

The CPU 10 acquires dentition data 11 a, jaw bone data 11 b and mastication data for respective patients, and stores this data in the RAM 11 (S3).

Next, the CPU 10 creates combined data by comnbining the dentition data 11a and jaw bone data 11 b stored in the RAM 11 (S4), and creates dental crown data indicating the dental crown that will repair the lost portion in the dentition indicated by the created combined data (S5). Furthermore, the processing routine that creates this dental crown data will be described in detail later.

The CPU 10 adds the created dental crown data to the lost portion in the combined data created in S4, and creates occlusion data that indicates the occlusion state of the dental crown indicated by the dental crown data with the opposing tooth of the opposite jaw on the basis of this combined data (S6). Furthermore, the processing routine that creates this occlusion data will also be described in detail later.

Next, on the basis of the created occlusion data, the CPU 10 calculates the implantation position of the artificial tooth root that supports this dental crown (S7). Furthermore, the processing routine that calculates this artificial tooth root implantation position will also be described in detail later. In cases where the CPU 10 acquires implantation position data that indicates the calculated artificial tooth root implantation position, the CPU 10 displays the completion of this implantation position calculation processing on the display 14 (S8), and ends the implantation position determining processing.

As was described above, the shape of the jaw bone beneath the mucous membrane can be sufficiently grasped, and artificial tooth root implantation positions that sufficiently avoid the nervous system and nearby toot roots can be determined, on the basis of the combined data created by combining the dentition data 11a and jaw bone data 11b peculiar to the patient.

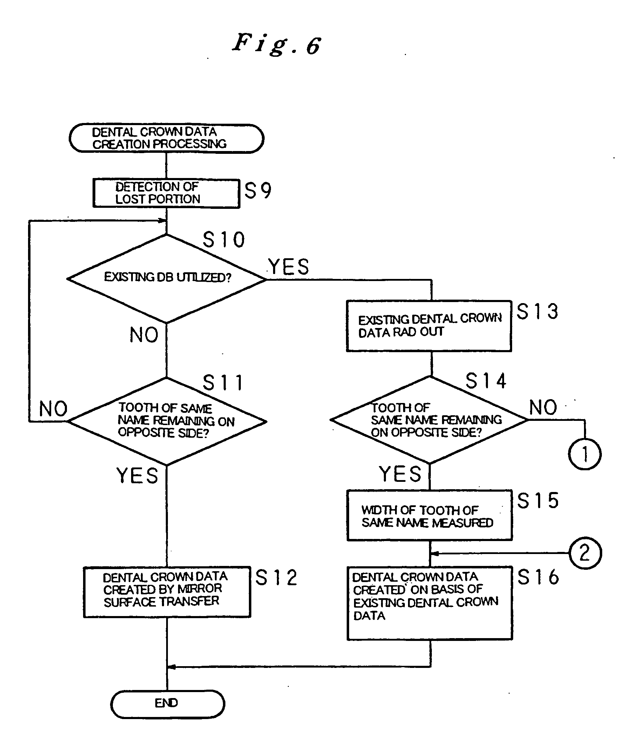

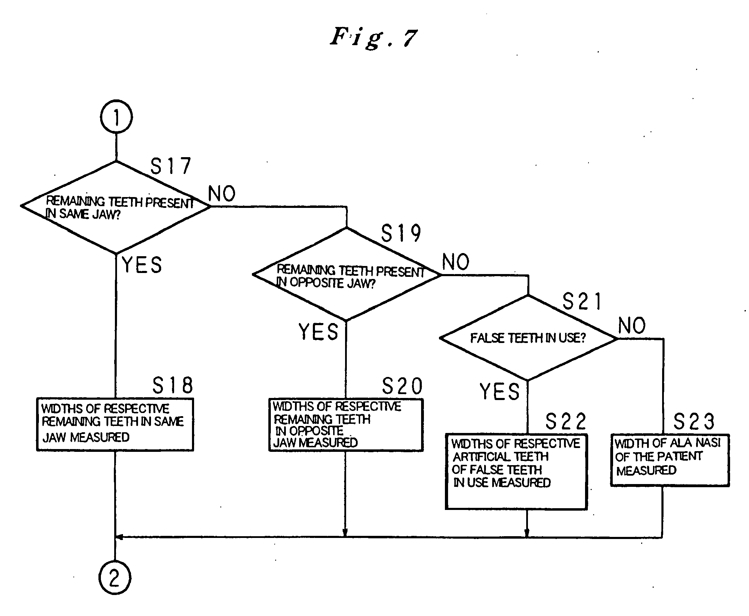

The dental crown data creation processing which is a subroutine in the abovementioned artificial tooth root implantation position determination processing (step S5 in Fig. 5) will be described below. Figs. 6 and 7 are flow charts showing the dental crown data creation processing routine performed by the computer 1.

On the basis of the combined data of the dentition data 11a and jaw bone data 11b created as described above, the CPU 10 detects lost portions in the respective dentitions of the upper and lower jaws indicated by the combined data (S9), and recognizes the position of any lost portion in the dentition of the upper jaw or lower jaw.

Next, in accordance with a selection made by the dental physician, the CPU 10 judges whether or not existing dental crown data in the existing DB stored in the HD 12 is to be utilized in the creation processing of the dental crown data indicating the dental crown that will repair the recognized lost portion [of the dentition] (S10), and in cases where it is judged that the existing DB will not be utilized (S10: NO), i. e., in cases where the dental crown data will be created on the basis of remaining teeth of the patient, a judgment is made as to whether or not a tooth of the same name on the opposite side in the same jaw with respect to the recognized lost portion remains (S11).

In concrete terms, a judgment is made as to whether or not a remaining tooth is present in the left-right symmetrical position (centered on the tooth positioned in the center) with respect to the recognized lost portion in the dentition (upper jaw or lower jaw) containing this recognized lost portion, and in cases where it is judged that a remaining tooth is present in this position (S11: YES), dental crown data indicating the dental crown that will repair the lost portion is created by the mirror image transfer of dental crown data for this remaining tooth (S12). Here, in cases where it is judged that a tooth of the same name on the opposite side in the same jaw with respect to the lost portion does not remain (S11: NO), dental crown data cannot be created on the basis of the remaining teeth of this patient himself; accordingly, the processing returns to step S10.

On the other hand, in cases where it is judged in step S 10 that the existing DB will be utilized (S 10: YES), the CPU 10 reads out the existing dental crown data corresponding to the position of the recognized lost portion from the DB of the HD 12 into the RAM 11 (S13). Furthermore, the CPU 10 judges whether or not a tooth of the same name on the opposite side in the same jaw with respect to the recognized lost portion remains (S 14), and in cases where it is judged that this tooth of the same name remains (S 14: YES), the width of this remaining tooth of the same name is measured (S 15). Furthermore, the CPU 10 creates dental crown data with the measured width by enlarging or reducing the existing dental crown data that is read out into the RAM 11 (S16).

Furthermore, in cases where it is judged that a tooth of the same name on the opposite side of the same jaw with respect to the lost portion does not remain (S14: NO), the CPU 10 judges whether or not any other remaining teeth are present in this same jaw (S17), and in cases where it is judged that other remaining teeth are present (S17: YES), the widths of the respective remaining teeth in this same jaw are measured (S 18), and the width of the dental crown that will repair the lost portion is calculated on the basis of the mean dental crown width of the respective adjacent teeth (Bolton index or the like); furthermore, dental crown data having the calculated width is created by enlarging or reducing the existing dental crown data that is read out into the RAM 11 (S16).

Furthermore, in cases where it is judged in step S 17 that there is no remaining tooth in the same jaw as the lost portion (S 17: NO), the CPU 10 judges whether or not remaining teeth are present in the opposite jaw from this lost portion (S 19), and in cases where it is judged that remaining teeth are present in the opposite jaw (S19: YES), the widths of the respective remaining teeth in the opposite jaw are measured (S20), the width of the dental crown that will repair the lost portion [of the dentition] is similarly calculated from the measured widths of the respective remaining teeth, and dental crown data having the calculated width is created by enlarging or reducing the existing dental crown data that is read out into the RAM 11 (S16).

Furthermore, in cases where it is judged in step S 19 that there are no remaining teeth in the opposite jaw from the lost portion (S19: NO), the CPU 10 judges whether or not there are artificial teeth currently in use by the patient (S21), and in cases where it is judged that there are artificial teeth in use (S21: YES), the widths of the respective artificial teeth among these artificial teeth in use are measured (S22), the width of the dental crown that will repair the lost portion is calculated on the basis of the measured widths of the respective artificial teeth, and dental crown data having the calculated width is created by enlarging or reducing the existing dental crown data read out into the RAM 11 (S16).

In cases where it is judged in step S21 that there are no artificial teeth currently in use, either (S21: NO), the CPU 10 measures the width of the ala nasi of the patient (S23), calculates the width of the dental crown that will repair the lost portion on the basis of the measured ala nasi width, and creates dental crown data having the calculated width by enlarging or reducing the existing dental crown data read out into the RAM 11 (S16).

Thus, by creating dental crown data indicating the dental crown that will repair the lost portion of the dentition on the basis of existing dental crown data accumulated in a DB prepared beforehand, it is possible to create dental crown data corresponding to race, facial silhouette shape and the like even in patients who have no remaining teeth or artificial teeth in use; furthermore, dental crown data can easily be created by enlarging or reducing existing dental crown data read out from the DB.

Furthermore, the width of the dental crown that will repair the lost portion [of the dentition] is calculated on the basis of the widths of remaining teeth present in the same jaw with respect to the lost portion, and even in cases where there are no remaining teeth in the same jaw, dental crown data that conforms to the teeth and jaw line of the individual patient can be created by calculating the width of the dental crown on the basis of the widths of remaining teeth in the opposite jaw. Furthermore, even in cases where there are no remaining teeth, dental crown data conforming to the dentition currently used by the individual patient can be created by creating dental crown shapes on the basis of the shapes of artificial teeth currently in use.

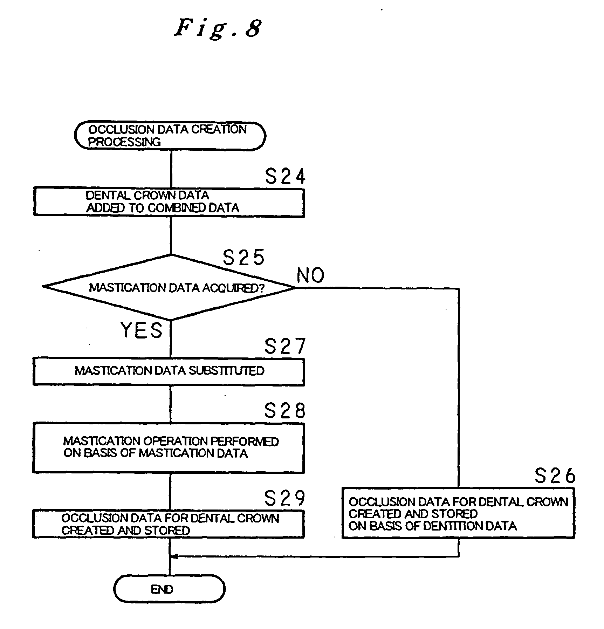

The occlusion data creation processing which is a subroutine of the abovementioned artificial tooth root implantation position determining processing (step S6 in Fig. 5) will be described below. Fig. 8 is a flow chart showing the occlusion data creation processing routine that is performed by the computer 1.

The CPU 10 adds the dental crown data created as described above to the combined data of the dentition data 11a and jaw bone data 11b (S24), thus creating combined data with no lost part, and judges whether or not mastication data has been acquired in step S3 in Fig. 5 (S25).

In cases where it is judged that mastication data has not been acquired (S25: NO), the CPU 10 causes appropriate occlusion of the respective opposing teeth of the upper jaw and lower jaw on the basis of the dentition data 11a in the combined data along the occlusion faces of the respective teeth indicated by this dentition data 11a, creates occlusion data that corrects the configuration of the occlusion face of the dental crown indicated by the dental crown data in this state with the opposing tooth in the central occlusion position (occlusion face engaging position), and stores this data in the RAM 11 (S26).

On the other hand, in cases where it is judged that mastication data has been acquired (S25: YES), the CPU 10 executes mastication movement (S28) based on the mastication data in virtual terms in the dentition indicated by the combined data by substituting the mastication data into this combined data (S27). As a result, the CPU 10 causes occlusion of the dental crown indicated by the dental crown data added to the combined data with the opposing tooth during the mastication movement that is peculiar to the individual patient, thus creating occlusion data that is adjusted to the central occlusion position and eccentric position (lateral movement), and stores this data in the RAM 11 (S29).

As was described above, dental crowns that are suited to the mastication operation peculiar to the individual patient can be formed by forming the occlusion face of the dental crown (that repairs the lost portion of the dentition) with the opposing tooth while taking into account the mastication data peculiar to the individual patient. Furthermore, even in cases where mastication data cannot be acquired, a dental crown with a mastication state adjusted to the current dentition of the individual patient can be reproduced by creating a dental crown with a shape that is based on the occlusion state of remaining teeth and artificial teeth currently in use in the dentition indicated by the combined data.

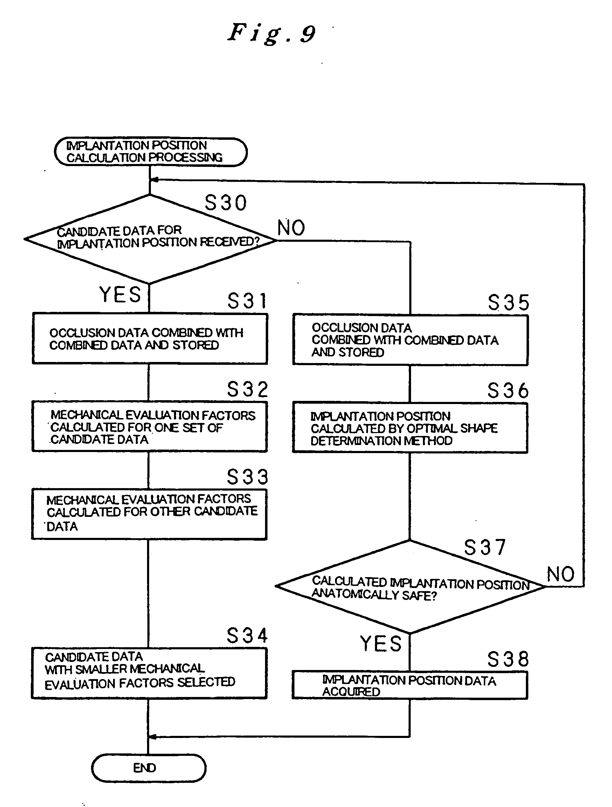

The artificial tooth root implantation position calculation processing which is a subroutine of the abovementioned artificial tooth root implantation position determining processing (step S7 in Fig. 5) will be described below. Fig. 9 is a flow chart showing the artificial tooth root implantation position calculation processing routine that is performed by the computer 1.

The CPU 10 judges whether or not implantation position candidate data has been received in order to determine the appropriate implantation position from the candidates input by the dental physician (S30), and in cases where it is judged that candidate data has been received (S30: YES), the implantation position data constituting the received candidate data is stored in the RAM 11. Furthermore, in the present embodiment, the computer 1 receives two sets of candidate data, and determines the implantation position by selecting one of the received sets of candidate data.

Furthermore, by combining the occlusion data created as described above with the combined data, the CPU 10 creates combined data which has an occlusion face that conforms to the opposing tooth in accordance with the occlusion data, and the CPU 10 stores this data in the RAM 11 (S31). Moreover, for one set of received implantation position candidate data, the CPU 10 calculates the mechanical evaluation factors generated in the jaw bone near the implantation position (indicated by this candidate data) as a result of the occlusion force based on the occlusion data (S32); similarly, for the other set of received implantation position candidate data, the CPU 10 calculates the mechanical evaluation factors generated in the jaw bone near the implantation position as a result of the occlusion force based on the occlusion data (S33).

The CPU 10 compares the values of the respective calculated mechanical evaluation factors, selects the candidate data showing the smaller values (S34), acquires the implantation position data indicating the artificial tooth root implantation position indicated by the selected candidate data, and stores this data in the RAM 11.

On the other hand, in cases where it is judged in step S30 that candidate data has not been received (S30: NO), i. e., in cases where the position in which the artificial tooth root is to be implanted is determined by the optimal shape determination method as chosen by the dental physician, the CPU 10 creates combined data having an occlusion face that conforms to the opposing tooth in accordance with the occlusion data by combining the occlusion data created as described above with the combined data, and stores this data in the RAM 11 (S35).

Furthermore, the CPU 10 calculates the mechanically optimal artificial tooth root implantation position in accordance with the optimal shape determination method (S36). In concrete terms, in a case where the sum of the mechanical evaluation factors obtained by altering the material characteristics of places where the mechanical evaluation factors generated on the basis of the occlusion data are large to the characteristics of the metal that is to be used as the artificial tooth root implantation position show a minimum value, the region where the material characteristics are altered to these metal characteristics is determined as the artificial tooth root implantation position.

The CPU 10 judges whether or not the implantation position thus calculated is anatomically safe (S37), and in cases where it is judged that this position is anatomically safe (S37: YES), the implantation position data indicating this implantation position is acquired and stored in the RAM 11 (S38). In concrete terms, the implantation angle and implantation distance (depth) of the artificial tooth root cavity used to implant the artificial tooth root that supports the dental crown indicated by the dental crown data are acquired.

Furthermore, in cases where the calculated implantation position is not anatomically safe (S37: NO), e. g., in cases where the calculated implantation position erodes the lower jaw channel beneath the mucous membrane in which the dental crown is to be implanted, the upper jaw cavity, adjacent tooth roots or the like, the processing returns to step S30. Here, since an appropriate implantation position cannot be calculated by implantation position calculation processing based on the optimal shape determination method performed by the computer 1 in cases where the implantation position calculated in step S36 is anatomically unsafe, the processing returns to step S30, a plurality of sets of implantation position candidate data that are anatomically safe in the judgment of the dental physician are input, and processing that determines a more appropriate implantation position from these sets of candidate data is executed by the computer 1.

In regard to artificial tooth root implantation positions calculated on the basis of occlusion data for the dental crown that supplements the lost portion of the dentition as described above, in cases where the values of the mechanical evaluation factors generated in the vicinity when artificial tooth roots are implanted in such implantation positions are small and mechanically stable, and [the positions] are anatomically safe, the shape of the jaw bone beneath the mucous membrane can be sufficiently grasped, and artificial tooth root implantation positions that sufficiently avoid the nervous system and nearby tooth roots can be specified, by determining these implantation positions.

Furthermore, on the basis of the implantation positions determined as described above, the shape of the artificial tooth root cavity bored in such implantation positions can be predicted; accordingly, the shape of the implant that is to be implanted can be predicted so that an implant of the desired shape can also be selected from existing structural bodies (artificial tooth roots).

The manufacturing processing of the guide member which is an auxiliary member used to perform precise boring of the artificial tooth root cavity in which the artificial tooth root is implanted on the basis of the artificial tooth root implantation position determined by the computer 1 as described above will be described below.

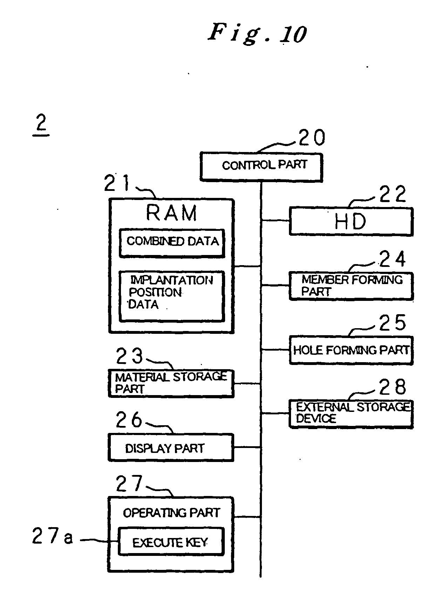

Fig. 10 is a block diagram which shows an example of the construction of the guide member manufacturing device of the present invention; in this figures, 2 indicates the guide member manufacturing device of the present invention. This guide member manufacturing device 2 is constructed from a personal computer or the like, and comprises a control part 20, RAM 21, HD 22, material storage part 23, member forming part (forming means) 24, hole boring part (hole boring means) 25, display part 26, operating part 27, external storage part 28 and the like.

In concrete terms, the control part 20 is constructed from a CPU or the like, and is connected to the abovementioned respective hardware parts of the guide member manufacturing device 2 via a bus; the control part 20 controls these hardware parts, and successively executes computer programs that are stored in the HD 22.

The HD 22 stores various types of computer programs that are necessary for the operation of the guide member manufacturing device 2 of the present invention.

The RAM 21 is constructed from an SRAM, DRAM, flash memory or the like, and stores temporary data that is generated during the execution of computer programs by the control part 20. Furthermore, in cases where a flash memory is used as the RAM 21, there is no loss of the stored contents even in cases where the power supply is cut off due to a power failure, movement of the guide member manufacturing device 2 or the like.

The external storage device 28 is constructed from a CD-ROM drive, flexible disk drive or the like; various types of data are read out from a portable recording medium such as a CD-ROM, flexible disk or the like on which various types of data and computer programs are recorded, and such data is stored in the HD 22.

The guide member manufacturing device 2 may have a construction in which this device 2 is connected via a cable (not shown in the figures) to the computer 1 used as the abovementioned artificial tooth root implantation position determining instrument, so that the combined data and implantation position data calculated by the computer 1 are acquired and stored in the HD 22 via this cable; alternatively, a construction may be used in which combined data and implantation position data that are temporarily recorded on a recording medium such as a CD-ROM, flexible disk or the like from the computer 1 are acquired and stored in the HD 22 via the external storage device 28. The control part 20 of the guide member manufacturing device 2 can use respective data in the guide member manufacturing processing by reading out combined data and implantation position data thus stored in the HD 22 into the RAM 21.

The material storage part 23 stores (for example) acrylic resin blocks or the like in which an acrylic resin is formed into an appropriate shape as the raw material of the guide member; such acrylic resin blocks are appropriately fed out to the member forming part 24 in accordance with instructions from the control part 20.

The member forming part 24 is constructed from a cutting tool or the like; in accordance with instructions from the control part 20, this part cuts acrylic resin blocks acquired from the material storage part 23 on the basis of dental crown data contained in the combined data stored in the RAM 21 as described above and data indicating teeth adjacent to the dental crown indicated by this dental crown data, thus forming [the acrylic resin] into the shapes of the dental crown and adjacent teeth, and feeds [the resulting formed members] out to the hole boring part 25. Furthermore, in the lost portions of the dentition of the dentition of the patient, the abovementioned cut acrylic resin blocks are mounted by being fastened to teeth adjacent to these lost portions; accordingly, the blocks are formed into a crown shape in the portions formed on the adjacent teeth.

The hole boring part 25 bores a guide hole (in a position that is connected to the implantation position data stored in the RAM 21) in accordance with instructions from the control part 20 in the acrylic resin block that has been cut into the desired shape by the member forming part 24.

The display part 26 is a display device such as a liquid crystal display (LCD) or the like, and displays the operating state of the guide member manufacturing device 2, information of which the dental physician is to be notified and the like.

The operating part 27 comprises function keys which are required in order to operate the guide member manufacturing device 2, an execute key 27a which is used to cause the guide member manufacturing device 2 to execute guide member manufacturing processing and the like. Furthermore, some or all of the various keys of the operating part 27 may be replaced by using a touch panel system for the display part 26.

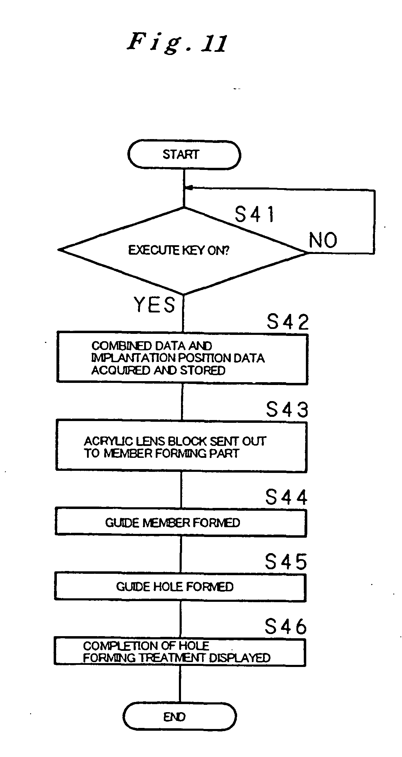

The guide member manufacturing processing performed by the guide member manufacturing device 2 constructed as described above will be described below. Fig. 11 is a flow chart which shows the guide member manufacturing processing routine that is performed by the guide member manufacturing device 2 of the present invention.

In cases where the guide member manufacturing device 2 is to be operated, the dental physician calculates the combined data ad implantation position data using the computer 1 as described above, and causes the guide member manufacturing device 2 to perform guide member manufacturing processing by switching the execute key 27a installed in the operating part 27 of the guide member manufacturing device 2 to "on".

In the guide member manufacturing device 2, the control part 20 judges whether or not the execute key 27a has been switched "on" (S41), and in cases where it is not judged that the execute key 27a has been switched "on" (S41: NO), the control part waits until this key is switched "on". In cases where it is judged that this key has been switched "on" (S41: YES), the respective computer programs stored in the HDD 22 are read out into the RAM 21 and successively executed, so that combined data and implantation position data are acquired from the outside and stored in the RAM 21 (S42)

Next, the control part 20 feeds out one acrylic resin block stored in the material storage part 23 to the member forming part 24 (S43), and cutting processing based on the combined data stored in the RAM 21 is executed by the member forming part 24 so that a guide member is formed by being cut into the shape of the dental crown (and adjacent remaining teeth) indicated by the dental crown data contained in the combined data (S44).

Next, the control part 20 causes the hole boring part 25 to bore a guide hole in a position that communicates with the implantation position (indicated by the implantation position data stored in the RAM 21) in the guide member formed in step S44, on the basis of the abovementioned implantation position data (S45).

As a result, in a case where the guide member is mounted in the procedure area of the patient, i.e., in concrete terms, in the lost portion of the dentition, by being fastened to the remaining teeth that are adjacent to this lost portion, the abovementioned guide hole is disposed in a position that is an extension of the artificial tooth root cavity that is to be bored.

In cases where a guide hole has been bored in the desired location of the guide member, the control part 20 displays [an indication of] the completion of the hole boring processing on the display part 26 (S46), and ends the guide member manufacturing processing.

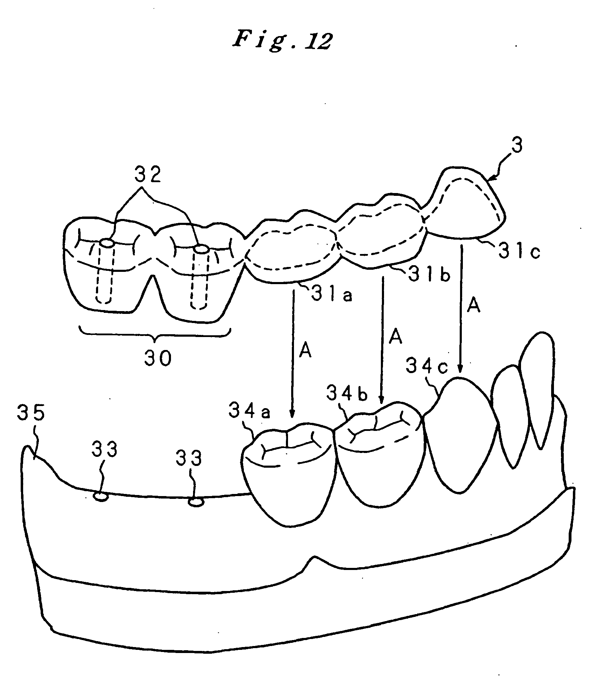

Fig. 12 is a model diagram showing one example of the guide member. In this figure, 3 indicates the guide member. The guide member 3 comprises a dental crown part 30 which is formed in the shape of the dental crown that supplements the lost portion of the dentition, and supporting parts 31 a, 31 band 31 c that are caused to cover the adjacent teeth 34a, 34b and 34c adjacent to the lost portion, and that support the dental crown part 30. Furthermore, guide holes 32, 32 are bored in the dental crown part 30.

In such a guide member 3, as is indicated by the arrow symbols A, A, A in the figures, the dental crown part 30 can be fastened by causing the supporting parts 31a, 31b and 31 c to cover the respective adjacent teeth 34a, 34b and 34c. In the case of such a guide member 3, the tip end portion of the hole boring device such as a drill or the like is engaged with the respective guide holes 32, 32, so that hole boring beneath the mucous membrane 35 is performed along the guide holes 32, 32, thus boring artificial tooth root cavities 33, 33 in the target implantation positions.

Furthermore, in the case of patients using a complete set of false teeth (so-called full dentures) or the like, there are no remaining teeth at all, so that the guide member 3 cannot be anchored; accordingly, a guide member with a shape in which the base surface of the dental crown part 30 agrees with the configuration of the surface of the jaw bone may be manufactured so that dental crown part 30 can mounted directly on the surface of the jaw bone.

Thus, by using a dental crown that repairs the dentition and a guide member 3 that covers the vicinity of this dental crown, [both] manufactured on the basis of an artificial tooth root implantation position calculated with good precision by the abovementioned computer 1, it is possible for guide holes 32, 32 to guide the tip end portion of the hole boring device during the hole boring procedure of the artificial tooth root cavities 33, 33, so that even a dental physician with little experience in the boring procedures of such artificial tooth root cavities 33, 33 can bore artificial tooth root cavities 33, 33 with good precision.

Furthermore, the raw material of the guide member is not limited to an acrylic block; the guide member can also be manufactured using a resin. Moreover, the manufacturing method is not limited to a manufacturing method using the abovementioned cutting treatment; the guide member can also be manufactured by molding using a mold. Furthermore, a guide member produced by cutting an acrylic block may also be subjected to resin polymerization using wax.

A sensor which is mounted in the hole boring device that bores the artificial tooth root cavities 33, 33 using a guide member 3 manufactured by the guide member manufacturing device 2 as described above, and which detects the hole boring direction of this hole boring device will be described below.

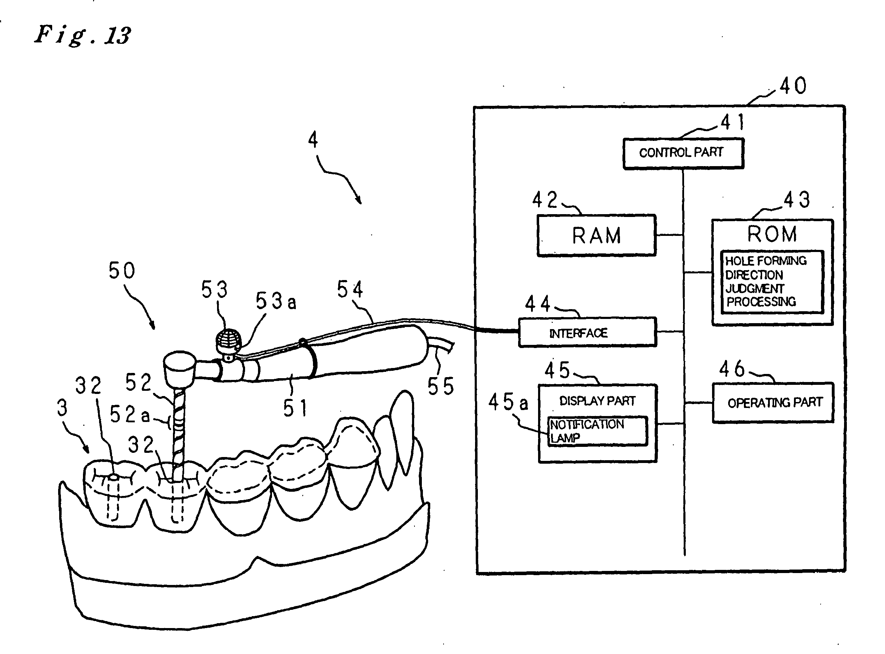

Fig. 13 is a block diagram which shows an example of the construction of the sensor of the present invention; in this figure, 4 indicates the sensor of the present invention. This sensor 4 is constructed by connecting a main body part 40 and a detection part (detection means) 53 constructed from a gyro sensor such as a silicon gyro, angle sensor or the like (mounted on a hole boring device 50) via a cable 54. Furthermore, a construction in which this main body part 40 and detection part 53 are formed as an integral unit is also possible.

The main body part 40 comprises a control part 41, RAM 42, ROM 43, display part 45, operating part 46, and interface 44 used for connection with the detection part 53.

In concrete terms, the control part 41 is constructed from a CPU, MPU or the like, and is connected with the abovementioned respective hardware parts of the main body part 40 via a bus, and with the detection part 53 via the interface 44; this control part 41 controls the abovementioned hardware parts, and successively executes computer programs that are stored in the RON 43.

The ROM 43 stores various types of computer programs that are necessary for the operation of the sensor 4 of the present invention, e. g., the hole boring direction judgment processing program. The RAM 42 is constructed from an SRAM, DRAM, flash memory or the like, and stores temporary data that is generated during the execution of computer programs by the control part 41.

The display part 45 is a display device such as a liquid crystal display or the like, and displays the operating state of the sensor 4, information of which the dental physician is to be notified, and the like. Furthermore, this display part 45 also comprises a notification lamp 45a consisting of an LED (light emitting diode) or the like used as notification means for providing notification of errors in the hole boring direction of the hole boring device 50. Furthermore, this notification lamp 45a is lit with (for example) a blue light if the hole boring direction of the hole boring device 50 is appropriate, and is lit with (for example) a red light if the hole boring direction is inappropriate. Moreover, instead of a notification lamp 45a, it would also be possible to use a construction comprising a buzzer or the like used to provide notification of errors in the hole boring direction of the hole boring device 50 by means of sound.

The operating part 46 comprises function keys or the like that are necessary in order to operate the sensor 4. Furthermore, some or all of the various keys of the operating part 46 may also be replaced by using a touch panel system for the display part 45.

The hole boring device 50 comprises a holding part 51 that can be sufficiently held by the dental physician; furthermore, the drill part (drill) 52 of the present invention is detachably attached to one end part of the holding part 51 perpendicular to the length direction of this holding part 51. Furthermore, a mark 52a formed by laser light is disposed on this drill part 52 in a position that is separated from the tip end portion (constituting the starting position of the hole boring treatment) by a distance based on the implantation position data that is calculated by the computer I acting as the abovementioned artificial tooth root implantation position determining instrument. Moreover, a mark 52a consisting of a thick line and a fine line in an appropriate position separated from the thick line is disposed on the drill part 52 shown in Fig. 13, and can be used as a visual indicator of the hole boring distance during the hole boring treatment. Furthermore, this drill part 52 is connected to an engine part (not shown in the figures) via a cable 55, and is constructed so that the drill can be caused to rotate by the supply of electric power from this engine part.

In the sensor 4 of the present embodiment, the detection part 53 is mounted in an appropriate position on the holding part 51 of the hole boring device 50 that allows detection of the hole boring direction of the drill part 52.

The detection part 53 inputs the detected hole boring direction of the drill part 52 of the hole boring device 50 into the control part 41 of the main body part 40 via the cable 54, and control part 41 operates as judgment means for judging whether or not the hole boring direction of the drill part 52 is appropriate by executing a hole boring direction judgment processing program that is stored in the ROM 43. Furthermore, a setting key 53a is disposed in the detection part 53, and the detection direction of the detection part 53 at the point in time at which this setting key 53a is switched "on" is stored in the RAM 42 of the main body part 40 as a set direction.

As is shown in Fig. 13, the hole boring device 50 in which the detection part 53 constructed as described above is mounted can maintain the hole boring direction of the drill part 52 by engaging the drill part 52 respectively along the guide holes 32, 32 of the guide member 53 mounted in the procedure area of the patient using the guide member 3 manufactured by the guide member manufacturing device 2. Furthermore, a hole boring treatment can be performed while securely maintaining an appropriate hole boring direction, as a result of the hole boring direction in the drill part 52 detected by the detection part 53 being calibrated beforehand by the setting key 53a in the main body part 40, and a judgment being made by the control part 41 as to whether or not this hole boring direction is the set direction stored in the RAM 42.

Furthermore, the detection part 53 may be constructed not only from a gyro sensor, but also from an optical sensor or the like, and can perform an artificial tooth root cavity boring treatment with better precision by providing this detection part 53 with a construction that allows the three-dimensional detection of the position of the arm of the dental physician, the position of the hole boring device 50, the physique of the patient, the position of the jaw and the like.

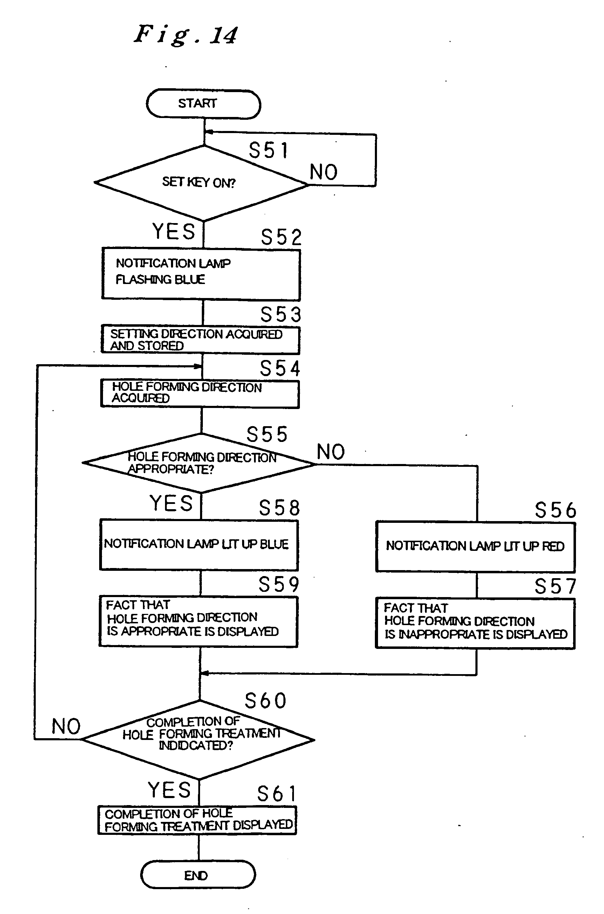

The artificial tooth root cavity boring processing that is performed by the hole boring device 50 in which the detection part 53 constructed as described above is mounted will be described below. Fig. 14 is a flow chart showing the artificial tooth root cavity boring processing routine that is performed by the hole boring device 50 in which the detection part 53 of the present invention is mounted.

As is shown in Fig. 13, the dental physician performing the artificial tooth root cavity boring procedure mounts the guide member 3 manufactured by the guide member manufacturing device 2 as described above in the procedure area of the patient, i. e., the lost portion of the dentition, holds the holding part 51 of the hole boring device 50, engages the tip end portion of the drill part 52 from the open ends of the guide holes 32 of the guide member 3 mounted in the procedure area of the patient, and switches the setting key 53a of the detection part 53 to "on".

The detection part 53 judges whether or not the setting key 53a has been switched "on" (S51), and in cases where it is not judged that the setting key 53a has been switched "on" (S51: NO), the detection part 53 waits until this key is switched "on". In cases where it is judged that this key has been switched "on" (S51: YES), the hole boring direction of the drill part 52 detected at this point in time is input into the main body part 40 via the cable 54.

In the main body part 40, the control part 41 causes the notification lamp 45a disposed in the display part 45 to flash with a blue color (S52), and at the same time, stores the hole boring direction acquired from the detection part 53 in the RAM 42 as the set direction (S53).

Meanwhile, the detection part 53 detects the hole boring direction of the drill part 52 at a specified timing under the control of the control part 41, and successively inputs this direction into the control part 41 of the main body part 40 via the cable 54.

The control part 41 successively acquires the hole boring direction of the drill part 52 detected by the detection part 53 (S54), and judges whether or not the hole boring direction acquired from the detection part 53 agrees with the set direction stored in the RAM 42 (and thus whether or not this direction is the appropriate hole boring direction) by reading out the hole boring direction judgment processing program stored in the ROM 43 into the RAM 42 and executing this program (S55). The guide holes 32 in the guide member 3 are bored so as to communicate with the artificial tooth root cavity that is to be bored, and it is necessary to engage the drill part 52 so that the central axis of the drill part 52 coincides with the central axes of the guide holes 32.

In cases where the control part 41 judges that the hole boring direction acquired from the detection part 53 is not appropriate (S55: NO), the control part 41 causes the notification lamp 45a to light up with a red light (S56), and displays an indication that the hole boring direction is inappropriate on the display part 45 (S57). Furthermore, the color with which the notification lamp 45a is lit may also be varied in accordance with the degree of inappropriateness of the hole boring direction of the drill part 52 by making it possible to light up this notification lamp 45a with three colors, i. e., blue, yellow and red. Moreover, besides such a notification lamp 45a, it would also be possible to use a construction that emits a warning sound as means for providing notification that the hole boring direction of the drill part 52 is inappropriate.

In cases where the dental physician confirms that the notification lamp 45a is lit with a red light, the dental physician stops the rotation of the drill part 52, and appropriately corrects the hole boring direction of the drill part 52.

On the other hand, in cases where it is judged that the hole boring direction acquired from the detection part 53 is appropriate (S55: YES), the control part 41 causes the notification lamp 45a to light up with a blue light (S58), and displays an indication that the hole boring direction is appropriate on the display part 45 (S59).

The dental physician recognizes the hole boring depth on the basis of the position of the mark 52a applied to the drill part 52, and in cases where hole boring has been performed to the hole boring depth indicated by the mark 52a, the dental physician can give an instruction to end the hole boring treatment by operating the operating part 46 or the like.

The control part 41 judges whether or not there has been an instruction from the dental physician to end the hole boring treatment (S60), and in cases where there has been no instruction to end the hole boring treatment (S60: NO), the control part 41 returns to the processing of step S54, and repeats the judgment processing as to whether or not the hole boring direction in the drill part 52 is appropriate, until ending [of the hole boring processing] is instructed.

Furthermore, in cases where it is judged that the ending of the hole boring processing of the artificial tooth root cavity has been instructed (S60: YES), the control part 41 displays an indication of the completion of the hole boring processing on the display part 45 (S61), and ends the processing.

Subsequently, the dental physician implants an artificial tooth root which is an existing structural body made of titanium in the bored artificial tooth root cavity, and can then implant a dental crown manufactured by the artificial tooth manufacturing device (described later) by connecting this dental crown to the protruding end part of this artificial tooth root, thus repairing the lost portion in the dentition, and implanting an artificial tooth root and dental crown more suited to the individual patient.

The processing in which the dental crown supported on the artificial tooth root is manufactured as described above on the basis of the dental crown data calculated by the computer 1 acting as an artificial tooth root implantation position determining instrument, and the occlusion data in the dental crown indicated by this dental crown data, will be described below.

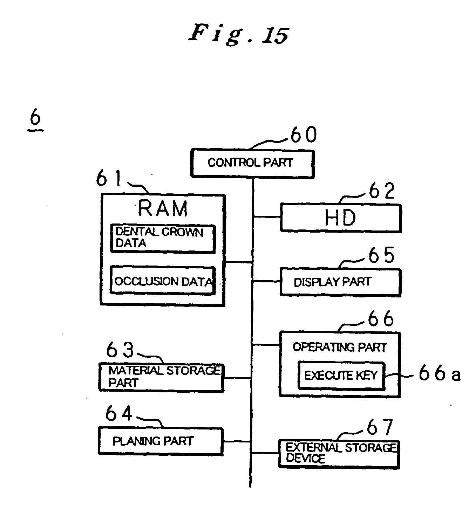

Fig. 15 is a block diagram showing an example of the construction of the artificial tooth manufacturing device of the present invention; in this figure, 6 indicates the artificial tooth manufacturing device of the present invention. This artificial tooth manufacturing device 6 consists of a personal computer or the like, and comprises a control part 60, RAM 61, HD 62, material storage part 63, grinding part (grinding means) 64, display part 65, operating part 66, external storage device 67 and the like.

In concrete terms, the control part 60 is constructed from a CPU or the like, and is connected to the abovementioned respective hardware parts of the artificial tooth manufacturing device 6 via a bus; this control part 60 controls these hardware parts, and successively executes computer programs stored in the HD 62.

The HD 62 stores various computer programs that are necessary for the operation of the artificial tooth manufacturing device 6 of the present invention.

The RAM 61 is constructed from an SRAM, DRAM, flash memory or the like, and stores temporary data that is generated during the execution of computer programs by the control part 60.

The external storage device 67 is constructed from a CD-ROM drive, flexible disk drive or the like; this storage device reads out various types of data from a portable recording medium such as a CD-ROM, flexible disk or the like on which various types of data and computer programs are recorded, and stores this data in the HD 62.

The artificial tooth manufacturing device 6 may have a construction in which this device is connected via a cable (not shown in the figures) to the computer 1 acting as the abovementioned artificial tooth root implantation position determining instrument, so that the dental crown data and occlusion data calculated by the computer 1 are acquired via this cable and stored in the HD 62, or may have a construction in which dental crown data and occlusion data that are temporarily recorded on a recording medium such as a CD-ROM, flexible disk or the like from the computer 1 are acquired via the external storage device 67 and stored in the HD 62. Furthermore, when artificial tooth manufacturing processing is executed, the control part 60 of the artificial tooth manufacturing device 6 reads out the dental crown data and occlusion data of the HD 62 into the RAM 61.

The material storage part 63 stores dental crown members in which (for example) titanium, ceramics, resins or the like used as dental crown (artificial tooth) raw materials are formed into appropriate shapes, and appropriately feeds out these members to the grinding part 64 under the control of the control part 60.

The grinding part 64 is constructed from a cutting tool or the like; under the control of the control part 60, this part 64 cuts dental crown members acquired from the material storage part 63 into shapes indicated by the dental crown data and occlusion data stored in the RAM 61 as described above.

The display part 65 is a display device such as a liquid crystal display or the like; this part 65 displays the operating state of the artificial tooth manufacturing device 6, information of which the dental physician is to be notified and the like.

The operating part 66 comprises function keys that are necessary in order to operate the artificial tooth manufacturing device 6, an execute key 66a which is used to cause the artificial tooth manufacturing device 6 to execute dental crown manufacturing processing, and the like. Furthermore, some or all of the various keys of the operating part 66 can be replaced by using a touch panel system for the display part 65.

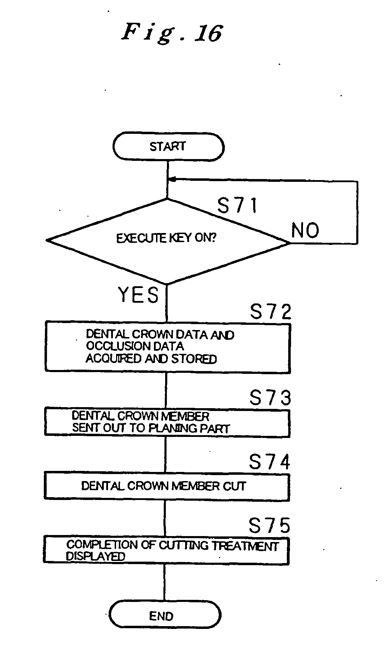

The dental crown manufacturing processing that is performed by the artificial tooth manufacturing device 6 constructed as described above will be described below. Fig. 16 is a flow chart which shows the dental crown manufacturing processing routine that is performed by the artificial tooth manufacturing device 6 of the present invention.

In cases where the artificial tooth manufacturing device 6 is to be operated, the dental physician acquires the dental crown data and occlusion data that are created when artificial tooth root implantation position determining processing is executed using the computer 1 as described above, and causes the artificial tooth manufacturing device 6 to execute dental crown manufacturing processing by switching the execute key 66a disposed in the operating part 66 of the artificial tooth manufacturing device 6 to "on".

In the artificial tooth manufacturing device 6, the control part 60 judges whether or not the execute key 66a has been switched "on" (S71), and in cases where it is not judged that the execute key 66a has been switched "on" (S71: NO), the control part 60 waits until this key is switched "on". In cases where it is judged that this key has been switched "on" (S71: YES), the control part 60 reads out the respective computer programs stored in the HD 62 into the RAM 61, and successively executes these programs, so that dental crown data and occlusion data are acquired from the outside and stored in the RAM 61 (S72).

Next, the control part 60 feeds out one dental crown member stored in the material storage part 63 to the grinding part 64 (S73), and cutting processing based on the dental crown data and occlusion data stored in the RAM 61 is executed by the grinding part 64, so that this dental crown member is cut into a dental crown which has the occlusion face indicated by the occlusion data, and the shape indicated by the dental crown data (S74).

When the cutting processing based on the dental crown data and occlusion data is ended, the control part 60 displays [an indication of] the completion of the cutting processing on the display part 65 (S75), and ends the dental crown manufacturing processing.

By manufacturing a dental crown that repairs the lost portion of the dentition using dental crown data and occlusion data calculated with good precision by the computer 1 as described above, it is possible to achieve effective utilization of the various types of data obtained in the calculation of an appropriate artificial tooth root implantation position; furthermore, an artificial tooth that conforms to the remaining teeth and artificial teeth currently in use in the dentition of the patient can be manufactured.

In the embodiment described above, the computer 1 acquires the computer programs that are used to cause operation as an artificial tooth root implantation position determining instrument from a recording medium via the external storage device 13, and stores these programs in the HD 12. However, it would also be possible to use a construction in which [these programs] are downloaded into the HD 12 beforehand. Furthermore, it would also be possible to use a construction in which respective computer programs are acquired from other communications devices via an external network by providing a communications interface or the like that is used to connect such a network to the computer 1.

### (Second Embodiment)

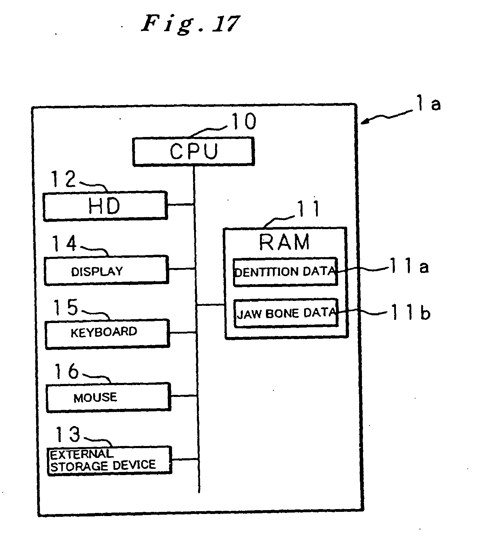

Fig. 17 is a block diagram showing an example of the construction of an artificial tooth root implantation position determining instrument constituting a second embodiment of the present invention. In Fig. 17, constituent members that are the same as in Fig. 1 are labeled with the same symbols, and a description of such constituent members is omitted.

Like the computer 1 of the abovementioned first embodiment, the computer 1a of the second embodiment combines dentition data 11a and jaw bone data 11b acquired from the outside, and creates dental crown data indicating a dental crown that will repair the lost portion of the dentition on the basis of this combined data.

Furthermore, in the computer 1a, the CPU 10 operates as candidate receiving means that receive a plurality of sets of candidate data (two sets of candidate data in the present embodiment) for the artificial tooth root implantation position from the dental physician on the basis of combined data in which created dental crown data is added to the lost portion. Furthermore, the CPU 10 operates as mechanical evaluation factor calculation means that calculate the mechanical evaluation factors generated in the jaw bone near the implantation positions indicated by the respective sets of candidate data for the two sets of candidate data that are received. Moreover, the CPU 10 operates as determining means for determining the candidate data in which the respective calculated mechanical evaluation factors are smaller as the implantation position.

Furthermore, in the present embodiment, for the combined data to which the created dental crown data has been added, occlusion data which is such that the occlusion faces in the respective teeth are flat is combined, and the mechanical evaluation factors generated in the vicinity of the respective implantation positions are calculated by applying a specified load to such occlusion faces.

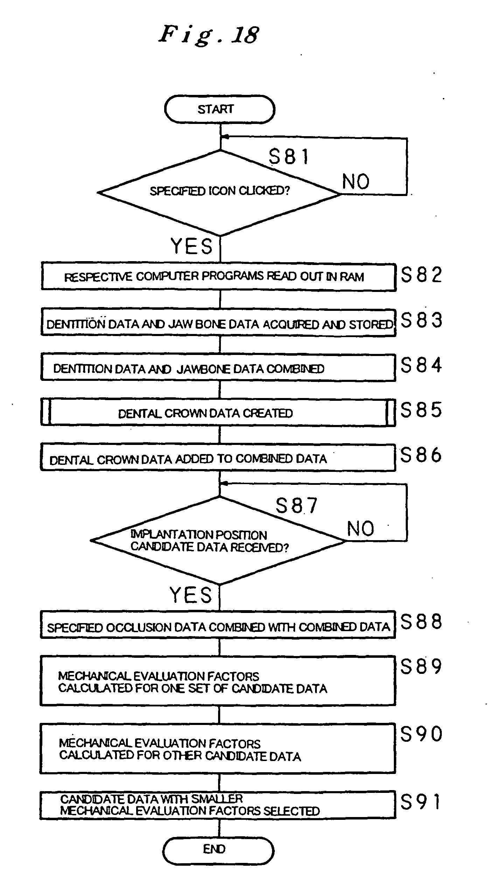

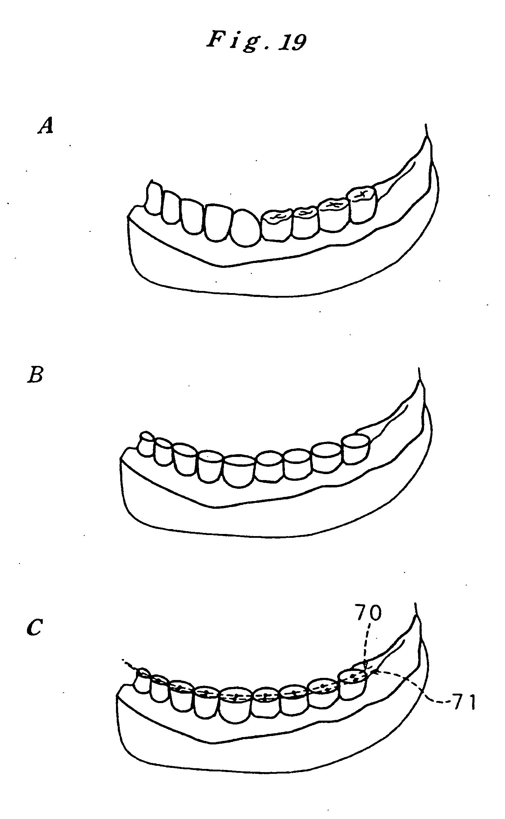

The artificial tooth root implantation position determining processing performed by the computer 1a constructed as described above will be described below. Fig. 18 is a flow chart showing the artificial tooth root implantation position determining processing routine performed by the computer 1a, and Fig. 19 is an explanatory diagram of the artificial tooth root implantation position determining processing routine performed by the computer 1a of the second embodiment.

When the computer 1a is to be operated as an artificial tooth root implantation position determining instrument, the dental physician acquires dentition data 11a and jaw bone data 11b for the subject patient using respective external devices, and causes the computer 1a to execute artificial tooth root implantation position determining processing by clicking on a specified icon disposed in the computer 1a using the mouse 16 or the like.

In the computer 1a, the CPU 10 judges whether or not the [abovementioned] specified icon has been clicked on (S81), and in cases where it is not judged that this icon has been clicked on (S81: NO), the CPU 10 waits until the specified icon has been clicked on. In cases where it is judged that this icon has been clicked on (S81: YES), the respective computer programs of the present invention stored in the HD 12 are read out into the RAM 11 (S82), and the program codes contained in the respective computer programs are successively executed.

First, the CPU 10 reads out the dentition data 11a and jaw bone data 11b for the individual patient that has been acquired from the outside and stored in the HD 12, and stores this data in the RAM 11 (S83). Then, the CPU 10 creates combined data by combining the stored dentition data 11a and jaw bone data 11b (S84). Furthermore, the CPU 10 creates dental crown data which indicates a dental crown that will repair the lost portion in the dentition indicated by the created combined data (S85). Moreover, the processing routine that creates this dental crown data is the same as the routine indicated in the first embodiment; accordingly, a description of this routine will be omitted.

Next, the CPU 10 adds the created dental crown data to the lost portion in the combined data created in step S84 (S86), thus creating combined data in which the lost portion is repaired by this dental crown data.

Here, in the second embodiment, the system is constructed so that the dental physician inputs two sets of candidate data into the computer 1 a as candidates for the artificial tooth root implantation position that is to be determined (on the basis of the abovementioned combined data), and the CPU 10 judges whether or not candidate data has been received from the dental physician (S87). In cases where it is judged that candidate data has not been received (S87: NO), the CPU 10 waits until such candidate data is received. In cases where it is judged that candidate data has been received (S87: YES), the received candidate data is stored in the RAM 11.

Furthermore, in step S86, the CPU 10 combines specified occlusion data with the combined data to which [the abovementioned] dental crown data has been added (S88), thus creating combined data in which the respective teeth have flat occlusion faces.

Here, Fig. 19 (a) is a model diagram of a portion of the dentition based on combined data in which the lost portion is repaired by dental crown data; combined data for dentition having a flat occlusion face as shown in Fig. 19 (b) is created by combining specified occlusion data with this combined data.

Furthermore, for one set of received implantation position data, the CPU 10 calculates the mechanical evaluation factors generated in the vicinity of this implantation position by a specified occlusion force (S89). Here, in a case where a specified occlusion force is applied perpendicularly in the respective positions indicated by the dots on the respective broken lines 70 and 71 in Fig. 19 (c) with respect to the occlusion planes of the respective teeth indicated by the occlusion data in combined data such as that shown in Fig. 19 (b), the mechanical evaluation factors generated in the vicinity of the implantation position indicated by this candidate data are calculated.

Furthermore, the calculation processing routine for these mechanical evaluation factors is the same as the routine indicated in the first embodiment. Moreover, the positions indicated by the broken line 70 in Fig. 19 (c) are the center positions of the occlusion faces of the respective teeth, and the positions indicated by the broken line 71 are (for example) positions that have an offset of approximately 3 mm toward the outside from the center positions; however, the positions to which the occlusion force is applied are not limited to such positions.

Similarly, for the other received set of candidate data for the implantation position, the CPU 10 calculates the mechanical evaluation factors generated in the jaw bone near the implantation position by a specified occlusion force (S90). The CPU 10 compares the respective calculated values of the mechanical evaluation factors, selects the candidate data showing the smaller values (S91), acquires the implantation position data that indicates the artificial tooth root implantation position indicated by the selected candidate data, and stores this data in the RAM 11.

Thus, on the basis of combined data created by combining three-dimensional dentition data 11a and jaw bone data 11b as described above, the computer 1a selects the more appropriate implantation position from the two implantation positions given by the dental physician as candidates after grasping the shapes of the jaw bone beneath the mucous membrane, the nervous system and the toot roots in the vicinity, thus making it possible to determine an implantation position that is reliably safe in anatomical terms and more stable in mechanical terms.

Furthermore, a guide member 3 which is an auxiliary member used in the artificial tooth root cavity boring procedure can be manufactured by the guide member manufacturing device 2 described in the first embodiment utilizing the combined data and implantation position data created when the computer 1a determines the artificial tooth root implantation position as described above. Furthermore, using this guide member 3, an artificial tooth root cavity boring procedure can be performed safely and with good precision by means of a hole boring device 50 to which the sensor 4 and drill part 52 described in the first embodiment are attached.

As was described above in detail, the present invention is a system for determining the implantation positions of artificial tooth roots (implants) for supporting artificial teeth that repair lost parts of the dentition. However, a similar anatomical and mechanical analysis can also be used in simulation operations in bone transplant procedures. Furthermore, the amount of bone to be transplanted, the shape of the implant that is to be implanted following transplantation, the implantation position and the like can also be determined on the basis of the predicted results obtained by simulation. Moreover, pattern molds, guide members and the like that are suited to the amount of bone thus determined by simulation can also be manufactured by computer processing (CAD/CAM) in order to aid in bone transplantation procedures.

Furthermore, [the present invention] is not limited to dental implants, but can also be applied to implantation position determining processing for biological implants including implants used in plastic surgery (surgical transplant materials, surgical implant materials) performed on the basis of mechanical analysis that takes into account external forces that are applied to various parts of the human body.

### INDUSTRIAL APPLICABILITY

As was described above in detail, the use of the artificial tooth root implantation position determining instrument of the present invention makes it possible to obtain a three-dimensional grasp of the shapes of the jaw bone beneath the mucous membrane, the shapes of the tooth roots of respective teeth and the like on the basis of combined data combining dentition data and jaw bone data acquired from respective patients; accordingly, artificial tooth root implantation positions that sufficiently avoid the lower jaw channel, upper jaw cavity and adjacent tooth roots can be determined.

Furthermore, since implantation positions calculated according to the finite element method have a high mechanical stability, mechanical evaluation factors generated in artificial tooth roots that are engaged in such implantation positions, and in the jaw bone in the vicinity of such implantation positions can be further reduced.

Moreover, artificial teeth that repair lost portions of the dentition can be formed on the basis of the remaining teeth and artificial teeth currently in use in respective patients, and an occlusion state of artificial teeth that conforms to the occlusion state of such remaining teeth and artificial teeth currently in use can be reproduced. An adjustment to suit other adjacent teeth can be maintained, and artificial teeth that produce no strange feeling can be formed. Furthermore, by forming artificial teeth on the basis of the mastication operation in the remaining teeth and artificial teeth currently in use in respective patients, it is possible to form artificial teeth that take into account the mastication operations of individual patients, and that have shapes showing greater conformity to other adjacent teeth.

Furthermore, guide members that are used in the artificial tooth root cavity boring procedure can be manufactured using the artificial tooth data and implantation positions created by the artificial tooth root implantation position determining instrument of the present invention. Moreover, in the case of hole boring operations utilizing such guide members, it is possible to perform the procedure even in cases where the dental physician has little experience in hole boring procedures by mounting a sensor that guides the hole boring direction along guide holes bored in the abovementioned guide member, and a drill with marks that serve as an indicator of the hole boring depth, in the hole boring device; furthermore, in the case of dental physicians with ample experience, the procedure can be caused to proceed safely and securely. Moreover, artificial teeth constituting the dental crown portions on implanted artificial tooth roots can also be manufactured with good precision utilizing the artificial tooth data and occlusion information created using the artificial tooth root implantation position determining instrument of the present invention.

## Claims

1. An artificial tooth root implantation position determining instrument for determining the implantation position of an artificial tooth root which supports an artificial tooth that supplements the lost portion of dentition, the instrument comprising:
dentition data acquisition means for acquiring three-dimensional data relating to said dentition (11a);
jaw bone data acquisition means for acquiring three-dimensional data relating to the jaw bone connectable to said dentition (11b);
combining means (1) for combining the three-dimensional data relating to dentition acquired by said dentition data acquisition means (11a) and the three-dimensional data relating to the jaw bone acquired by said jaw bone data acquisition means (11b);
artificial tooth data creating means (13a) for creating artificial tooth data indicating an artificial tooth that supplements the lost portion of said dentition indicated by the combined data for dentition and jaw bone created by said combining means;
candidate receiving means (10) for receiving a plurality of candidates for the implantation position of said artificial tooth root on the basis of combined data to which artificial tooth data created by said artificial tooth data creating means (13a) is added; and
determining means for determining one implantation position from the candidates received by said candidate receiving means.

2. The artificial tooth root implantation position determining instrument according to claim 1, wherein the instrument comprises mechanical evaluation factor calculating means for calculating the mechanical evaluation factors generated in the respective vicinities by a preset occlusion force for the implantation positions respectively indicated by each of the candidates received by said candidate receiving means, and said determining means are constructed so that the implantation position in which the mechanical evaluation factor calculated by said mechanical evaluation factor calculating means shows a minimum value is determined.

3. The artificial tooth root implantation position determining instrument according to claim 2, wherein said three-dimensional data relating to the jaw bone includes hardness information for said jaw bone, and said mechanical evaluation factor calculating means are constructed so that said mechanical evaluation factors are calculated on the basis of said hardness information.

## Patentansprüche

1. Implantationspositionsbestimmungsinstrument zur Bestimmung der Implantatsposition einer künstlichen Zahnwurzel, die einen künstlichen Zahn trägt, der den verlorenen Teil eines Gebisses ergänzt, wobei das Instrument Folgendes aufweist:
ein Gebissdatengewinnungsmittel zum Gewinnen dreidimensionaler Daten (11a), die sich auf das Gebiss beziehen;
Kieferknochendatengewinnungsmittel zum Gewinnen dreidimensionaler Daten (11b), die sich auf den Kieferknochen beziehen, der mit dem Gebiss verbunden werden kann;
Kombinationsmittel (1) zum Kombinieren der dreidimensionalen Daten (11a), die sich auf das Gebiss beziehen, die vom Gebissdatengewinnungsmittel gewonnen wurden, und der dreidimensionalen Daten (11b), die sich auf den Kieferknochen beziehen, die vom Kieferknochendatengewinnungsmittel gewonnen wurden,;
Mittel (13a) zum Erstellen von Daten für einen künstlichen Zahn zum Erstellen von Daten für einen künstlichen Zahn, die einen künstlichen Zahn angeben, der den verlorenen Teil des Gebisses ergänzt, der durch die kombinierten Daten für ein Gebiss und einen Kieferknochen angegeben wird, die von dem Kombinationsmittel erstellt werden;
Kandidatempfangsmittel (10) zum Empfangen mehrerer Kandidaten für die Implantatsposition der künstlichen Zahnwurzel auf der Basis von kombinierten Daten, zu welchen Daten für einen künstlichen Zahn, die von dem Mittel (13a) zum Erstellen von Daten für einen künstlichen Zahn erstellt werden, hinzugefügt werden; und
Bestimmungsmittel zum Bestimmen einer Implantatsposition aus den Kandidaten, die von dem Kandidatempfangsmittel empfangen werden.

2. Implantatspositionpositionsbestimmungsinstrument einer künstlichen Zahnwurzel nach Anspruch 1, wobei das Instrument Berechnungsmittel für einen mechanischen Auswertungsfaktor aufweist zum Berechnen der mechanischen Auswertungsfaktoren, die in den entsprechenden Nahbereichen durch eine voreingestellte Gebissschlusskraft erzeugt werden, für die Implantatspositionen, die jeweils von jedem der Kandidaten angegeben werden, die von dem Kandidatempfangsmittel empfangen werden, und wobei das Bestimmungsmittel so konstruiert ist, dass die Implantatsposition, in der der mechanische Auswertungsfaktor, der von dem Berechnungsmittel für einen mechanischen Auswertungsfaktor berechnet wird, einen Minimalwert zeigt, bestimmt wird.

3. Implantatspositionpositionsbestimmungsinstrument zur Bestimmung der Implantatsposition einer künstlichen Zahnwurzel nach Anspruch 2, wobei die dreidimensionalen Daten, die sich auf den Kieferknochen beziehen, Härteinformationen für den Kieferknochen enthalten und das Berechnungsmittel für einen mechanischen Auswertungsfaktor so konstruiert ist, dass die mechanischen Auswertungsfaktoren auf der Basis der Härteinformationen berechnet werden.

## Revendications

1. Instrument de détermination de position d'implantation de racine de dent artificielle destiné à déterminer la position d'implantation d'une racine de dent artificielle qui supporte une dent artificielle qui remplace la partie de dentition perdue, l'instrument comprenant :
des moyens d'acquisition de données de dentition pour acquérir des données tridimensionnelles relatives à ladite dentition (11a) ;
des moyens d'acquisition de données de mâchoire pour acquérir des données tridimensionnelles relatives à la mâchoire liables à ladite dentition (11b) ;
des moyens de combinaison (1) pour combiner les données tridimensionnelles relatives à ladite dentition acquises par lesdits moyens d'acquisition de données de dentition (11a) et les données tridimensionnelles relatives à la mâchoire acquises par lesdits moyens d'acquisition de données de mâchoire (11b) ;
des moyens de création de données de dent artificielle (13a) pour créer des données de dent artificielle indiquant une dent artificielle qui remplace la partie perdue de ladite dentition indiquée par les données combinées de dentition et de mâchoire créées par lesdits moyens de combinaison ;
des moyens de réception de candidats (10) pour recevoir une pluralité de candidats pour la position d'implantation de ladite racine de dent artificielle sur la base de données combinées auxquelles des données de dent artificielle créées par ledit moyen de création de données de dent artificielle (13a) sont ajoutées ; et
des moyens de détermination pour déterminer une position d'implantation à partir des candidats reçus par lesdits moyens de réception de candidats.

2. Instrument de détermination de position d'implantation de racine de dent artificielle selon la revendication 1, dans lequel l'instrument comprend un moyen de calcul de facteur d'évaluation mécanique pour calculer les facteurs d'évaluation mécanique générés dans les voisinages respectifs par une force d'occlusion prédéfinie pour les positions d'implantation respectivement indiquées par chacun des candidats reçus par ledit moyen de réception de candidats, et lesdits moyens de détermination sont construits de manière à ce que la position d'implantation dans laquelle le facteur d'élévation mécanique calculé par lesdits moyens de calcul de facteur d'évaluation mécanique présente une valeur minimale soit déterminée.

3. Instrument de détermination de position d'implantation de racine de dent artificielle selon la revendication 2, dans lequel lesdites données tridimensionnelles relatives à la mâchoire comprennent des informations de dureté pour ladite mâchoire, et lesdits moyens de calcul de facteur d'évaluation mécanique sont construits de manière à ce que lesdits facteurs d'évaluation mécanique soient calculés sur la base desdites informations de dureté.
